(19) Europäisches Patentamt
European Patent Office
Office européen des brevets

(11) EP 3 950 123 B1

(12) **EUROPEAN PATENT SPECIFICATION**

(45) Date of publication and mention of the grant of the patent:
**10.04.2024 Bulletin 2024/15**

(21) Application number: **19934376.5**

(22) Date of filing: **10.12.2019**

(51) International Patent Classification (IPC):
***B01J 31/02*** *(2006.01)* ***C07C 68/06*** *(2020.01)*
***C07C 69/96*** *(2006.01)*

(52) Cooperative Patent Classification (CPC):
(C-Sets available)
**B01J 31/0292; B01J 31/0279; B01J 31/0282; B01J 31/0298; C07C 68/06;** B01J 2231/49 (Cont.)

(86) International application number:
**PCT/CN2019/124322**

(87) International publication number:
**WO 2021/114091 (17.06.2021 Gazette 2021/24)**

(54) **HETEROGENEOUS CATALYST BASED ON IONIC LIQUID, AND PREPARATION METHOD THEREFOR AND USE THEREOF**

HETEROGENER KATALYSATOR AUF BASIS VON IONISCHER FLÜSSIGKEIT, HERSTELLUNGSVERFAHREN DAFÜR UND VERWENDUNG DAVON

CATALYSEUR HÉTÉROGÈNE À BASE DE LIQUIDE IONIQUE, ET PROCÉDÉ DE PRÉPARATION ASSOCIÉ ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2019 CN 201911253627**

(43) Date of publication of application:
**09.02.2022 Bulletin 2022/06**

(73) Proprietors:
- **Shenyang University of Chemical Technology Liaoning 110142 (CN)**
- **Shinghwa Advanced Material Group Co., Ltd Shandong 257503 (CN)**

(72) Inventors:
- **SHI, Lei Shenyang Liaoning 110142 (CN)**
- **GUO, Jianjun Dongying Shandong 257503 (CN)**
- **LI, Xin Dongying Shandong 257503 (CN)**
- **LIU, Mingyu Shenyang Liaoning 110142 (CN)**

(74) Representative: **Ter Meer Steinmeister & Partner Patentanwälte mbB Nymphenburger Straße 4 80335 München (DE)**

(56) References cited:

| | |
|---|---|
| **CN-A- 102 617 365** | **CN-A- 102 675 028** |
| **CN-A- 102 863 339** | **CN-A- 102 863 339** |
| **CN-A- 102 978 012** | **CN-A- 103 521 263** |
| **CN-A- 103 641 720** | **CN-A- 104 311 407** |
| **CN-A- 106 582 813** | **CN-A- 107 162 879** |
| **CN-A- 108 117 536** | **CN-A- 109 593 170** |
| **CN-A- 109 593 170** | **CN-B- 102 863 339** |

- **CHEN XUEWEI ET AL: "Solvent-free aza-Markovnikov and aza-Michael additions promoted by a catalytic amount of imidazolide basic ionic liquids", TETRAHEDRON LETTERS, vol. 52, no. 28, 1 July 2011 (2011-07-01), pages 3588-3591, XP55922222, Amsterdam , NL ISSN: 0040-4039, DOI: 10.1016/j.tetlet.2011.04.117**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 950 123 B1

- **DATABASE CA [Online] CHEMICAL ABSTRACTS SERVICE, COLUMBUS, OHIO, US; PAN, SU-JUAN ET AL: "Synthesis of di-2-ethy hexyl carbonate using [Bmim]Im as a catalyst", XP002806544, retrieved from STN Database accession no. 2011:885985 & PAN, SU-JUAN ET AL: "Synthesis of di-2-ethy hexyl carbonate using [Bmim]Im as a catalyst", DALIAN GONGYE DAXUE XUEBAO , 30(2), 129-132 CODEN: DGDXAP; ISSN: 1674-1404, 2011,**

(52) Cooperative Patent Classification (CPC): (Cont.)

C-Sets
**C07C 68/06, C07C 69/96**

## Description

## TECHNICAL FIELD

The present disclosure belongs to the field of chemistry and chemical engineering, and relates to synthesis of high-purity electrolyte for lithium ion battery, in particular to synthetic method of a new ionic liquid with strong alkalinity, an embedding-riveting immobilized method of the new ionic liquid, and use of prepared heterogeneous catalyst and heterogeneous catalyst for catalytic synthesis of ethyl methyl carbonate.

## BACKGROUND

Ethyl Methyl Carbonate (EMC) is a linear carbonate compound with asymmetric structure, has the characteristics of both dimethyl carbonate (DMC) and diethyl carbonate (DEC), is miscible with almost all organic solvents such as alcohols, ethers and ketones and the like, and can be used as carbonylation reagent or alkylation reagent for organic synthesis reaction. EMC has high oxygen content, low toxicity and low persistent accumulation in organisms, has low solubility in water, is considered as an environment-friendly chemical, is a promising gasoline additive, can improve the octane value of gasoline, can replace methyl tert-butyl ether (MTBE), and can reduce the emission of solid particles and nitrogen oxides in automobile exhaust. Meanwhile, EMC has a flash point of 26.7°C which is far higher than the average flash point (-20°C) of the gasoline. EMC added into the gasoline can obviously improve the safety of storage and transportation. EMC is more importantly and widely used as excellent electrolyte solvent for lithium ion battery. EMC has small steric hindrance and asymmetry in terms of structure, and is easy to generate a double-ion structure together with lithium ion, which is equivalent to solvation lithium ion and further assists in increasing the solubility of the lithium ion and improving the capacitance density and electric quantity of the battery. EMC has excellent thermal conductivity and low resistance in terms of physical property. The battery has low self-heating amount and can timely dissipate heat during use, and thus the heat cannot be accumulated in the battery to cause spontaneous combustion so that the safety of the lithium ion battery is improved. EMC has structural stability in electrochemical environment in terms of chemical property, and can generate solvation-like intermediate together with lithium ion, which is very stable and not easy to decompose to generate combustible gas (such as methane), and can increase the charging and discharging times of the battery, thereby prolonging the service life of the battery. EMC has a solidifying point of -14°C and a boiling point of 107°C, and can be used as solvent for widening the liquid phase range of the electrolyte, improving conductivity of the electrolyte in a low-temperature region and improving the low-temperature service performance of the battery, thereby facilitating widening the working temperature range of the electrolyte.

The synthetic method of EMC mainly comprises phosgenation, oxidative carbonylation, transesterification and the like. However, the battery grade EMC is mainly prepared with the transesterification. The sodium methoxide with strong alkalinity catalyst is often used in industry, and the product separation is realized by catalytic distillation. Sodium methoxide has a higher catalytic activity, but is sensitive to water and easy to inactivate. The sodium salt generated due to sodium methoxide participating in reaction has lower solubility in carbonate and is easy to separate out, resulting in that the catalyst cannot be reused and is difficult to be separated. A small amount of sodium salt residue would result in reduced purity of produced EMC, and the generated solid waste with strong alkalinity would pollute the environment.

Some scholars studied other strong organic alkali such as sodium ethoxide or sodium tert-butoxide and the like, soluble strong alkali including potassium hydroxide or sodium hydroxide and the like, and the medium alkali or weak alkali such as potassium carbonate, sodium carbonate or potassium fluoride and the like. However, soluble and strong organic/inorganic alkali has the drawbacks of quick inactivation, and generation of salt inactivation products which are insoluble in carbonate.

Ionic Liquids have characteristics of low vapor pressure, strong solubility, good thermal stability, reusability and the like, and thus are widely paid attention to. The research of EMC synthesis at present mainly focuses on alkyl imidazolium ionic liquids, including imidazolium ionic liquids with different anions and cations structures such as 1,3-dimethylimidazolium chloride ([Mmim]Cl), 1-ethyl-3- methylimidazolium chloride ([Emim]Cl), 1-butyl-3-methylimidazolium chloride ([Bmim]Cl), 1-ethyl-3-methylimidazolium bromide ([Emim]Br), 1-butyl-3-methylimidazolium bromide ([Bmim]Br), 1-butyl-2- ethyl-3-methylimidazolium bromide [Bemim]Br, 1-butyl-2-propyl- 3-methylimidazolium bromide ([Bpmim]Br), 1,3-dimethylimidazolium iodide ([Mmim]I), 1-ethyl-3-methylimidazolium iodide ([Emim]I), 1-butyl-3-methylimidazolium iodide ([Bmim]I), 1-butyl-3-methylimidazolium butyrate ([Bmim] $CH_3(CH_2)_2COO$) and (1-(4-hydroxy) butyl-3-methylimidazolium benzoate ([OHBmim]PhCOO) and the like. However, alkyl imidazolium salt ionic liquids for synthesizing EMC disclosed in literatures at present have the defects of low activity, long reaction time, high reaction concentration and the like, of which fundamental reason is weak nucleophilicity of halogen or acetate anion.

Literatures or patents further disclosed homogeneous lewis acid catalysts such as nitrates, lanthanum-containing soluble salts, titanates and organotin compounds, for example $Mg(NO_3)_2$, $La(NO_3)_3$, $LaCl_3$, $Ti(OBu)_4$, $Ti(OPh)_4$ or $Bu_2SnO$ and the like. However, during the transesterification of carbonate, the homogeneous catalysts can slowly

decompose and release products such as $NO_x$, $Cl^{-1}$ or butanol and the like, thereby resulting in reduced purity of the ethyl methyl carbonate.

In view of many disadvantages of homogeneous catalysts, catalysts for synthesizing ethyl methyl carbonate is gradually shifted to heterogeneous catalysts, for example, ion exchange resins, metal oxides and composite oxides such as MgO, CaO, $MgO-Al_2O_3$, Mg-Al-O-t-Bu or AlPO and the like, and alkaline molecular sieves or MOFs materials, such as Al-Zn-MCM-41, ZIF-8 or ZIF-67 and the like.

Among Lewatit K1221, Purofine PFC-100H, Dowex Marathon C, Dowex Marathon MR-3, Amberlyst 119 Wet, Lewatit K1131 and Lewatit K1461 in the sulfonic acid resins with strong acidity, Lewatit K1221 has the highest catalytic activity. Under the transesterification conditions that nDMC/nEtOH =1/2, reaction was carried out at 75°C for 26.7 h, and the catalyst is 6.75wt% of the total weight of the reaction raw materials, DMC conversion efficiency of 54% and EMC yield of 53% were achieved, wherein the reaction time was too long, and the efficiency was not high. Resin with super strong acidity, Nafion SAC-13, is a copolymer of tetrafluoroethylene and perfluoro-3,6-dioxa-4-methyl-7-octenesulfonyl fluoride. The sulfonyl group therein has super strong acidity due to strong electron-withdrawing capability of -CF2CF2SO3H group, so that the resin has high catalytic activity with DMC conversion efficiency of 61% and a EMC yield of 52%, which is close to that of Lewatit K1221. Although the ion exchange resin with strong alkalinity has high catalytic activity, the hydroxyl group thereof is easy to lose and thus the catalyst is quickly deactivated, which is similar to potassium hydroxide-like catalyst. Both resin catalysts cannot be used industrially.

Yang Yanzhao et al synthesized the $\gamma$-$Al_2O_3$ catalyst by precipitation method with $AlCl_3 \bullet 6H_2O$ and ammonia water as main raw materials. Under the condition that the loading amount of $Al_2O_3$ was 12wt%, the adding amount of the catalyst was 7wt%, nDMC/nDEC =1/1 and the reaction was carried out at 120°C for 8h, EMC yield of 63.6% could be achieved, in view of which the reaction temperature was too high and the catalytic efficiency was low. Shen et al researched that self-made solid alkalis such as MgO, ZnO, $La_2O_3$, $CeO_2$ and the like were used for transesterification of DMC and DEC in gas-phase and liquid-phase systems respectively. Under the condition that the reaction was carried out at 103°C for 4h and the adding amount of the catalyst was 2.4wt%, EMC yield was 44.2%, 26.5%, 12.6% and 7.3%, respectively, in view of which the activity of the catalyst could not meet the requirements of industrial application. Chen Ying et al synthesized $MgO-Al_2O_3$ composite metal oxide by co-precipitation method with NaOH and $Na_2CO_3$ as precipitating reagents, and found that the catalytic activity of the catalyst $MgO-Al_2O_3$ was superior to that of commercially available MgO and CaO. Under the condition that nDMC/nDEC =1/1 and the reaction was carried out at 103°C for 4 h, EMC yield of 45.8% could be achieved. Wang et al[58] synthesized mesoporous magnesium aluminate spinel $MgAl_2O_4$ (MAO) catalyst by evaporation-induced self-assembly method with aluminum isopropoxide and magnesium nitrate as main raw materials. Reaction condition were as follows: nDMC/nDEC =1/1, the reaction was carried out at 103°C for 0.5h, and the adding amount of the catalyst was 5wt%. Chen et al[59] synthesized an acid-alkali bifunctional mesoporous material $MgO-Al_2O_3$-SBA-15 by mixing unbaked SBA-15 (mesoporous silica), magnesium nitrate with aluminum nitrate, grinding them and then baking at high temperature. Under the condition that $n_{DMC}/n_{DEC}$=1/1, the reaction was carried out at 104°C for 4h and the adding amount of $MgO-Al_2O_3$-SBA-15 catalyst was 4 wt%, EMC yield of 46.1% was achieved. Wang et al[32] prepared magnetic Mg-Fe, a binary composite oxide catalyst, by co-precipitation method. Under the condition that nDMC/nDEC=1/1, the reaction was carried out at 100°C for 1.5h, and the adding amount of MgFe-400 catalyst was 1wt%, EMC yield of 51% was achieved. Miao et al[60] synthesized bimetallic Co/Zn-ZIF precursor (ZIF is the zeolite imidazolate framework) by "one-pot method". Under the condition that $n_{DMC}/n_{DEC}$=1/1, the reaction was carried out at 100°C for 7h and the adding amount of ZnCo/NC-600 catalyst was 1.0 wt%, EMC yield of 51.5% was achieved. Mei et al[17] prepared Mg-Al-$NO_3$ HT catalyst by co-precipitation method with NaOH as precipitating reagent and nitrate as precursor, then prepared Mg-Al-O-t-BuHT catalyst by stirring Mg-Al-$NO_3$ HT in tetrahydrofuran solution of potassium tert-butoxide for 24h, prepared Mg-Al-$CO_3$ HT catalyst by urea hydrolysis method, and prepared Mg-Al HT catalyst by calcining Mg-Al-$CO_3$ HT at 500°C for 5h. Under the condition that $n_{DMC}/n_{EtOH}$=1/5, the reaction was carried out at 80°C for 7h and the adding amount of the Mg-Al-O-t-BuHT catalyst was 1wt%, DMC conversion efficiency of 86.4% and EMC selectivity of 25.9% were achieved, and further the DMC conversion efficiency (87.9%) and EMC selectivity (24.3%) remained basically unchanged after the same catalyst was reused five times. Palani et al [26] synthesized a series of MCM-41 catalysts (Si/Al=50, Si/Al+Zn=100) by hydrothermal method with sodium metasilicate as silicon source, aluminum sulfate and zinc sulfate as main raw materials. A fixed-bed was used to evaluate the catalyst. Under the condition that $n_{DMC}/n_{DEC}$=1/1, a flow rate for raw materials was 1.5 mL/h, the adding amount of the catalyst was 0.5g (weight hourly space velocity for mixed raw material was $3.0h^{-1}$) and a reaction was carried out at 175°C, Al-Zn-MCM-41 (50) was found to have the best activity, with DMC conversion efficiency of 88% and EMC yield of 85%. Zhou et al[30] synthesized zeolite imidazole framework ZIF-8 ($[Zn(MeIm)_2]$, MeIm refers to 2-methylimidazole) with 2-methylimidazole, ammonia water and $Zn(OH)_2$ as main raw materials. Under the condition that $n_{DMC}/n_{DEC}$=1/1, the reaction was carried out at 100°C for 3h, and the adding amount of catalyst was 1wt%, ZIF-8 showed the best catalytic effect with EMC yield of 50.7%. Yang et al[31] synthesized zeolite imidazole framework ZIF-67 ($Co(MeIm)_2$) with 2-methylimidazole and cobalt nitrate hexahydrate as main raw materials. Under the condition that $n_{DMC}/n_{DEC}$=1/1, the reaction was carried out at 100°C for 24h and the adding amount of the catalyst of 2wt%, EMC yield of 50.32% was

achieved with the ZIF-8 as catalyst, while EMC yield was up to 83.39% with ZIF-6 as catalyst having a higher catalytic activity.

CHEN Xuewei et al (Solvent-free aza-Markovnikov and aza-Michael additions promoted by a catalytic amount of imidazolide basic ionic liquids", Tetrahedron letters , vol. 52, no. 28, pages 3588-3591) relates to the synthesis of a family of imidazolide ionic liquids. These ionic liquids combined the virtues of strong basicity and relatively good thermal stability. Catalytic properties of these imidazolide ionic liquids were investigated and satisfactory yield was achieved when 2.0 mol % of [Bmim]Im was used as catalyst for aza-Markovnikov addition under solvent-free condition at room temperature in one hour.

PAN Su-Juan et al (Synthesis of di-2-ethy hexyl carbonate using [Bmim]Im as a catalyst" DALIAN GONGYE DAXUE XUEBAO, 30(2), 129-132 CODEN: DGDXAP; ISSN: 1674-1404, 2011) relates to the preparation of di-2-ethy hexyl carbonate (DEHC) by transesterifying dimethyl carbonate (DMC) with 2-ethylhexanol (EHOH) in the presence of [Bmim]Im(paired by 1-butyl-3-methylimidazolium cation and imidazolide anion) as a catalyst.

CN103521263A relates to a morpholine salt ionic liquid catalyst and a preparation method thereof as well as a method for preparing biodiesel by use of morpholine salt ionic liquid catalyzing waste oil. The catalyst is prepared by the following steps of synthesizing an intermediate through a reaction between N-methylmorpholine and halogenated alkane; synthesizing morpholine salt through a reaction between morpholine and an alcoholic solution of inorganic base or salt; preparing the morpholine salt ionic liquid catalyst through a reaction between the intermediate and the morpholine salt. The biodiesel is prepared by taking the waste oil and alkyl alcohol as raw materials and the morpholine salt ionic liquid as a catalyst.

CN108117536A relates to a method for synthesizing propylene carbonate by virtue of 1,2-propanediol and carbon dioxide. The method comprises the steps of blending, and reacting said components at a reaction temperature of 150-180° C and a $CO_2$ pressure of 1MPa-10MPa for 24 hours with a basic ionic liquid as a catalyst and acetonitrile as a solvent, wherein the mass ratio of the catalyst to 1,2-propanediol is 0.01-0.04, and the mass ratio of the solvent to 1,2-propanediol is 4-8.

CN107162879A relates to a method for synthesizing nonylphenol from nonene and phenol through catalysis with an alkaline liquid. Compared to the traditional preparation method, the preparation method of the present invention has advantages of environmental protection, no corrosion to equipment, low catalyst consumption, rapid reaction, high product quality, easy separation of the product and the ionic liquid catalyst, low reaction temperature, and the like.

CN102863339B relates to a method for synthesizing methylethyl carbonate by ester exchange of dimethyl carbonate and diethyl carbonate, which is implemented by carrying out synthetic reaction of methylethyl carbonate on raw materials, dimethyl carbonate and diethyl carbonate by using an imidazole ionic liquid as a catalyst at certain reaction temperature under ordinary pressure for some reaction time.

CN106582813A relates to an immobilized ionic liquid catalyst for synthesizing ethyl methyl carbonate through an ester exchange reaction and a preparation method of the immobilized ionic liquid catalyst. According to the preparation method, immobilization of basic ionic liquid on a graphene oxide can be achieved at the temperature of 0-20 °C with graphene oxide as a carrier and a dilute NaOH solution as a solvent.

The heterogeneous catalysts disclosed in literatures or patents at present all have the following drawbacks: high reaction temperature which is generally higher than 100 °C, and longer reaction time which is generally longer than 5h. The fundamental reason thereof is the low catalytic activity.

The process for preparing ethyl methyl carbonate by catalytic distillation of dimethyl carbonate and ethanol needs to meet the requirements that an azeotrope of dimethyl carbonate and ethanol is taken from the column top at 63.4-63.6°C, the temperature of the column bottom was kept at about 73-75 °C, and it took 20-30 min for raw materials to fall into the column bottom from the column top through the catalytic distillation column. Therefore, a heterogeneous catalyst to be developed requires to exhibit high catalytic activity at 63-80°C, and a reaction equilibrium requires to be reached within 30 min. However, the activity of the heterogeneous catalyst disclosed by literatures and patents is significantly lower than the requirement of industrial application.

## SUMMARY

According to one aspect of the present disclosure, a heterogeneous catalyst as defined in claim 1 is provided. Aiming at the problems such as weak alkalinity and poor nucleophilicity of ionic liquids, poor stability and easy inactivation of ionic liquids with strong alkalinity and the like disclosed in the literatures or patents, the purpose of the present disclosure is to develop a new ionic liquid with strong alkalinity and having special structure and high-temperature resistant and high stability. A series of developed ionic liquids with strong alkalinity are used for synthesizing high-purity ethyl methyl carbonate product by transesterification between dimethyl carbonate and ethanol, transesterification between dimethyl carbonate and diethyl carbonate, or transesterification between methanol and diethyl carbonate, and exhibit extremely high reaction activity. A reaction equilibrium can be reached when the reaction time is 5 min and the reaction temperature is in a range from 76°C to 78°C, and the ionic liquids with strong alkalinity show high catalytic

activity even at near room temperature 30°C. The synthesized ionic liquids exhibit substantially unchanged catalytic activity and show higher stability, even after reused 20 times.

The prepared ionic liquids with strong alkalinity directly replace soluble strong alkali such as sodium hydroxide or potassium hydroxide to facilitate hydrolysis of single solution of ethyl orthosilicate, tetrabutyl titanate or aluminum silicate and the like, or hydrolysis of any mixed solution of thereof. The ionic liquid is embedded, by means of inlay, in silica, titanium dioxide, aluminum oxide or single/multiple oxygen-containing compound framework(s) with specific structure(s), without introducing Na+ or K+ ions at all. The prepared heterogeneous catalyst does not need to be baked, and can be directly applied to transesterification after being drying/vacuum drying, and exhibits excellent catalytic activity. Because the ionic liquid matrix has a double-nitrogen ring structure and can be embedded in (like a double-headed mountaineering cone) the framework of -Si-O-, -Ti-O-, -Al-O- or oxygen-containing composite compound, the immobilized heterogeneous catalyst shows higher stability, and the active component of ionic liquid with strong alkalinity is not easy to lose.

The heterogeneous catalyst comprises an ionic liquid and a support which embeds the ionic liquid in a riveting manner.

The ionic liquid comprises an anion and a cation; wherein the cation has a structure represented by Formula I or Formula II;

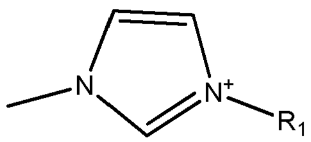 Formula I 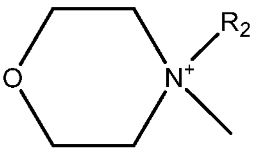 Formula II

the anion has a structure represented by Formula III, Formula IV or Formula V;

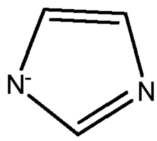 Formula III 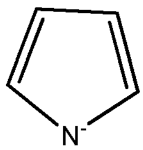 Formula IV 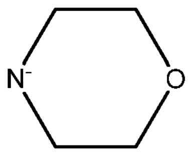 Formula V

wherein $R_1$ and $R_2$ independently from each other are C1-C6 alkyl, C2-C6 alkenyl or C3-C6 aryl.

Optionally, the ionic liquid is embedded in the framework of the support.

Optionally, at least one action of coupling and hydrogen bonding exists between the support and the ionic liquid.

Optionally, $R_1$ and $R_2$ independently from each other are $-CH_3$, $-CH_2CH_3$, $-(CH_2)_2CH_3$, or $-(CH_2)_3CH_3$.

Optionally, a weight content ratio of the active ionic liquid to the support in the heterogeneous catalyst is in a range of 1%-30%: 10%-60%.

Optionally, the support is at least one selected from silica, titanium dioxide, and aluminum oxide, and the ionic liquid is embedded in the framework of the support during the preparation of the support.

Optionally, the heterogeneous catalyst further comprises a structure regulator, and the ionic liquid is embedded in a composite framework of the structure regulator and the support. The structure regulator has a valence of 2, 3 or 4, and is used to construct a framework structure of composite oxide(s) together with the support, so as to improve the environment where the ionic liquid is embedded, thereby enhancing acting force between the ionic liquid and the composite oxide of support.

Optionally, the structure regulator comprises at least one selected from Mg, Ca, Ba, La, Ce, Zr, Fe, Zn, Li, Cs, and Al.

Optionally, a weight content of the structure regulator is in a range from 10% to 60%.

According to further aspect of the present disclosure, a method for preparing the heterogeneous catalyst is provided and comprises the following steps:

a) obtaining a homogeneous catalyst; wherein the homogeneous catalyst is prepared by the method comprising the following steps:

a1) adding an alkali into solution I comprising an ionic liquid anion source, and reacting to obtain an ionic liquid anion metal salt; and

a2) dissolving the ionic liquid anion metal salt in a solvent, adding an ionic liquid cation salt, and reacting to obtain the ionic liquid;

and

b) adding water to a mixture comprising a support precursor and an ionic liquid, and carrying out hydrolysis to obtain the heterogeneous catalyst,

wherein

in step a1), the ionic liquid anion source comprises imidazole, pyrrole or morpholine; in step a2), the ionic liquid cation salt is at least one selected from 1-$R_1$-3-methylimidazolium bromide, 1-$R_1$-3-methyl-imidazolium iodide, N-methyl-N-$R_2$-morpholinium bromide, and N-methyl-N-$R_2$-morpholinium iodide;

in step a2), the solvent comprises a water-carrying agent which is at least one selected from ethanol, benzene, toluene and xylene.

Optionally, in step a1), the solvent in the solution I is at least one selected from ethanol, benzene, toluene, and xylene; the alkali is an organic alkali or an inorganic alkali, wherein the organic alkali comprises sodium methoxide, sodium ethoxide or sodium tert-butoxide, potassium methoxide, potassium ethoxide or potassium tert-butoxide, and the inorganic alkali comprises sodium hydroxide or potassium hydroxide; and the ionic liquid anion metal salt is at least one selected from ionic liquid anion sodium salts and ionic liquid anion kalium salts.

Optionally, in step a1), the concentration of the ionic liquid anion source in the solution I is in a range from 0.05 g/mL to 0.8 g/mL; and a molar ratio of the ionic liquid anion source to the alkali is in a range from 0.9 to 1.1.

In step a1), the ionic liquid anion source comprises imidazole, pyrrole or morpholine.

Optionally, in step a1), reaction conditions are as follows: the reaction temperature is in a range from 50°C to 80°C and the reaction time is in a range from 5h to 12h.

Optionally, step a1) further comprises: after the reaction is completed, removing the solvent in the reaction system to obtain imidazole anion salt, pyrrole anion salt or morpholine anion salt.

In step a2), the solvent comprises a water-carrying agent which is at least one selected from ethanol, benzene, toluene and xylene; and the ionic liquid cation salt is at least one selected from 1-$R_1$-3-methyl-imidazolium bromide, 1-R1-3-methyl-imidazolium iodide, N-methyl-N-$R_2$-morpholinium bromide and N-methyl-N-$R_2$-morpholinium iodide.

Optionally, in step a2), the ratio of the ionic liquid anion metal salt to the solvent is in a range of 0.1-0.9: 0.05-1.2 g/mL.

Optionally, in step a2), reaction conditions are as follows: the reaction temperature is in a range from 25°C to 80°C and the reaction time is in a range from 12h to 48h.

Optionally, step a2) further comprises: after the reaction is completed, removing the solvent in the reaction system to obtain the ionic liquid.

As an embodiment, step a) comprises:

(1) dissolving commercially available imidazole, pyrrole or morpholine in a certain amount of a solvent such as ethanol, benzene, toluene, xylene and the like; slowly adding equimolar organic or inorganic strong alkali into a three-neck flask, wherein the inorganic strong alkali comprises NaOH and KOH, and the organic strong alkali comprises sodium methoxide, sodium ethoxide, sodium tert-butoxide and the like, or potassium methoxide, potassium ethoxide, potassium tert-butoxide and the like; carrying out vigorous stirring, wherein the stirring temperature is in a range from 50°C to 80°C and the stirring time is in a range from 5h to 12h; after the reaction is completed, carrying out rotary evaporation under the condition of reduced pressure and a temperature ranging from 60 °C to 120 °C in an oil bath to remove the solvent and alcohols or water produced; then drying in a vacuum drying oven until no weight change occurred to obtain the imidazole salt, the pyrrole salt or the morpholine salt, wherein the obtained imidazole salt, the pyrrole salt or the morpholine salt has sodium or kalium cation, namely the imidazole sodium or kalium salt, the pyrrole sodium or kalium salt and the morpholine sodium or kalium salt;

(2) dissolving a certain amount of imidazole salt, pyrrole salt or morpholine salt having sodium or kalium cation in a certain amount of water-carrying agent such as ethanol, benzene, toluene, xylene and the like, adding equimolar commercially available ionic liquid of 1-R-3-methylimidazolium bromide/iodide or N-methyl-N-R-morpholinium bromide/iodide into a three-neck flask to perform cation reaction, and carrying out vigorous stirring to obtain Na/KBr or Na/KI precipitate and target ionic liquid, wherein the stirring temperature is in a range from 25°C to 80°C and the stirring time is in a range from 12h to 48h; and

(3) filtering the mixture comprising the target ionic liquid many times, carrying out rotary evaporation under the condition of reduced pressure and a temperature ranging from 65°C to 100°C with a time ranging from 1h to 4h in an oil bath to remove the solvent, and drying in a vacuum drying oven until no weight change occurred to obtain a target product with pure components.

The imidazole-based, pyrrole-based or morpholine-based ionic liquid has the following structure, wherein the

cation is 1-R-3-methyl-imidazolium or N-methyl-N-R-morpholinium, R is a normal or isomeric structure of alkane, alkene and arene, the anion is imidazole, pyrrole or morpholine anion.

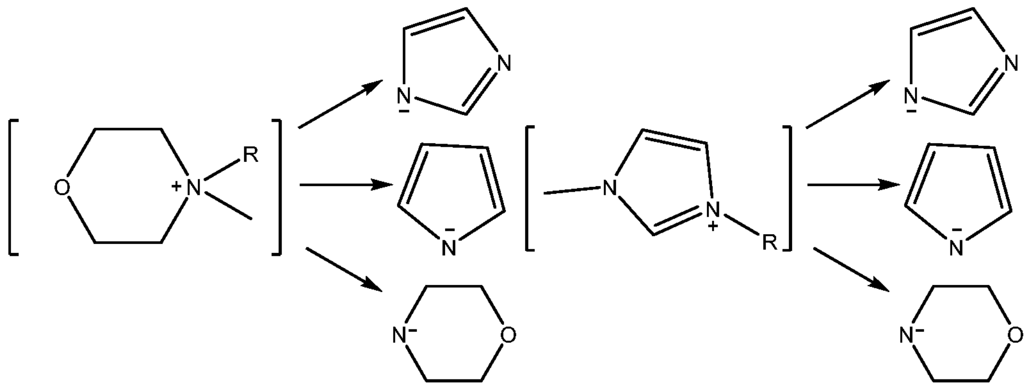

R = $-CH_3$, $-CH_2CH_3$, $-(CH_2)_2CH_3$, $-(CH_2)_3CH_3$ or other structure of alkane, alkene and arene

Optionally, the homogeneous catalyst is prepared with any of methods described above.

Optionally, in step b), the support precursor comprises at least one selected from ethyl orthosilicate, tetrabutyl titanate, aluminum isopropoxide, and sodium aluminate.

Optionally, the mixture in step b) further comprises a solvent, wherein the adding amount of the solvent is in a range from 0.1 to 0.9, and the solvent comprises at least one selected from methanol, ethanol, propanol, butanol, methyl acetate, and ethyl acetate.

Optionally, in step b), a weight ratio of the support precursor, ionic liquid and water is in a range of 0.2-0.8: 0.03-0.4: 0.1-0.4.

Optionally, in step b), after hydrolysis, an aging step is carried out, washing and drying steps are carried out to obtain the heterogeneous catalyst, wherein, the aging temperature is in a range from 40°C to 120°C and the aging time is in a range from 6h to 24h.

Optionally, in step b), conditions of the drying are as follows: vacuum drying temperature is in a range from 100°C to 150°C and the vacuum drying time is in a range from 2h to 6h.

Optionally, step b) further comprises: mixing the support precursor, the structure regulator precursor with the solvent, adding the ionic liquid therein, mixing uniformly, and then adding water to carry out hydrolysis to obtain the heterogeneous catalyst.

Optionally, in step b), the structure regulator precursor comprises at least one selected from corresponding acetate, silicate, hydrochloride, and nitrate of the structure regulator.

Optionally, in step b), a weight ratio of the support precursor, the structure regulator precursor, the solvent, the ionic liquid, and water is in a range of 0.2-0.8:0.05-0.4:0.3-0.8:0.03-0.4:0.1-0.4.

Optionally, step b) comprises: mixing uniformly the support precursor, the structure regulator with the solvent, then adding a certain amount of the above single or multiple ionic liquids therein, and after mixing uniformly, adding 0.8-1.5 times water of the calculated theoretical amount, controlling the temperature during hydrolysis to be in a range from 25°C to 80°C to form uniform gel product, wherein the ionic liquid serves as both a promoter of hydrolysis and an active component in the final heterogeneous catalyst, and after aging of the gel product at a temperature ranging from 40°C to 120°C and a time ranging from 6h to 24h, rinsing the resulting solid product with solvents of alcohols or esters 3 times to 5 times to dissolve the ionic liquid attached to the surface thereof, and finally, drying in vacuum at a drying temperature ranging from 100°C to 150°C and a drying time ranging from 2h to 6h to obtain heterogeneous catalyst with strong alkalinity and having the ionic liquid embedded-riveted therein.

According to further aspect of the present disclosure, a method for preparing ethyl methyl carbonate by transesterification is provided, characterized in that, the catalyst is an ionic liquid or an ionic liquid-based heterogeneous catalyst; wherein,

the ionic liquid comprises an anion and a cation; wherein, the cation has a structure represented by Formula I or Formula II;

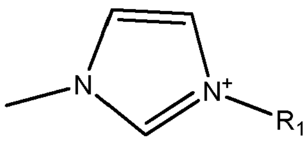 Formula I 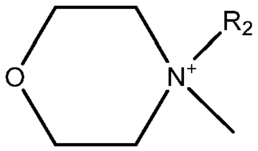 Formula II

the anion has a structure represented by Formula III, Formula IV or Formula V;

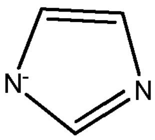 Formula III 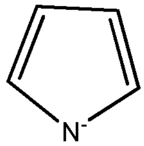 Formula IV 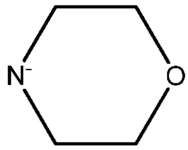 Formula V

wherein $R_1$ and $R_2$ independently from each other are C1-C6 alkyl, C2-C6 alkenyl or C3-C6 aryl; and
the ionic liquid-based heterogeneous catalyst is at least one selected from the heterogeneous catalysts described above.

Optionally, the transesterification comprises at least one selected from transesterification between dimethyl carbonate and ethanol, transesterification between dimethyl carbonate and diethyl carbonate, and transesterification between methanol and diethyl carbonate.

The conditions of the transesterification are as follows: a reaction equilibrium is reached when the reaction time is 5 min and the reaction temperature is in a range from 76°C to 78°C.

In the present disclosure, "[EmIm]Br" refers to 1-ethyl-3-methylimidazolium bromide. In the present disclosure, "[EmIm]Im" refers to 1-ethyl-3-methylimidazolium imidazole salt.

In the present disclosure, "[EmIm]py" refers to 1-ethyl-3-methylimidazolium pyrrole salt.

In the present disclosure, "EtOH" refers to ethanol.

In the present disclosure, "DMC" refers to dimethyl carbonate.

In the present disclosure, "EMC" refers to ethyl methyl carbonate.

In the present disclosure, "DEC" refers to diethyl carbonate.

In the present disclosure, C1-C6 refers to the number of carbon atoms contained. For example, the term "C1-C6 alkyl" refers to alkyl containing 1-6 carbon atoms.

In the present disclosure, the "alkyl" is a group formed from an alkane compound molecule by losing any one of hydrogen atoms. The alkane compound comprises straight-chain alkanes, branched-chain alkanes, cycloalkanes and branched-chain cycloalkanes.

In the present disclosure, the "alkenyl" is a group formed from an olefin compound molecule by losing any one of hydrogen atoms.

In the present disclosure, an "aryl" is a group formed from an aromatic compound molecule by losing one hydrogen atom on an aromatic ring; for example, the p-tolyl is formed from toluene by losing the hydrogen atom at the para-position with respect to the methyl group on the phenyl ring.

The beneficial effects achieved by the present disclosure are as follows:

1) The heterogeneous catalyst provided in the present disclosure is used for synthesizing high-purity ethyl methyl carbonate products by transesterification between dimethyl carbonate and ethanol, transesterification between dimethyl carbonate and diethyl carbonate, or transesterification between methanol and diethyl carbonate, and exhibit extremely high reaction activity. A reaction equilibrium can be reached when the reaction time is 5 min and the reaction temperature is in a range from 76°C to 78°C, and the heterogeneous catalyst shows high catalytic activity even at near room temperature 30°C;
2) The heterogeneous catalyst provided in the present disclosure exhibit substantially unchanged catalytic activity and show higher stability, even after reused 20 times; and
3) For the heterogeneous catalyst provided in the present disclosure, the prepared ionic liquids with strong alkalinity is used to directly replace soluble strong alkali such as sodium hydroxide or potassium hydroxide to facilitate hydrolysis of single solution of ethyl orthosilicate, tetrabutyl titanate or aluminum silicate and the like, or hydrolysis of any mixed solution of thereof. The ionic liquid is embedded, by means of inlay, in silica, titanium dioxide, aluminum oxide or single/multiple oxygen-containing compound framework(s) with specific structure(s), without introducing Na+ or K+ ions at all. The prepared heterogeneous catalyst does not need to be baked, and can be directly applied to transesterification after being dried/vacuum dried, and exhibits excellent catalytic activity. Because the ionic liquid matrix has a double-nitrogen ring structure and can be embedded in (like a double-headed mountaineering cone)

the framework of -Si-O-, - Ti-O-, -Al-O- or oxygen-containing composite compound, the immobilized heterogeneous catalyst shows higher stability, and the active component of ionic liquid with strong alkalinity is not easy to lose.

## BRIEF DESCRIPTION OF THE DRAWINGS

FIG. 1 is 1HNMR spectrum (Hydrogen-1 Nuclear Magnetic Resonance Spectrum) of [EmIm]Im ionic liquid synthesized by Example 1.

FIG.2 is 13CNMR spectrum (Carbon-13 Nuclear Magnetic Resonance Spectrum) of [EmIm]Im ionic liquid synthesized by Example 1.

FIG.3 is 1HNMR spectrum of [EmIm]py ionic liquid synthesized by Example 1.

FIG.4 is 13CNMR spectrum of [EmIm]py ionic liquid synthesized by Example 1.

FIG. 5 is photo of the prepared fresh 1-butyl-3-methylimidazolium pyrrole salt ionic liquid.

FIG. 6 is photo of 1-butyl-3-methylimidazolium pyrrole salt after reused 20 times.

FIG. 7 shows how the catalytic activity of ionic liquids varies with reused times.

FIG. 8 shows immobilized catalyst of 1-ethyl-3-methylimidazolium imidazole ionic liquid prepared by impregnating method.

FIG. 9 shows immobilized catalyst of 1-ethyl-3-methylimidazolium imidazole ionic liquid prepared by embedding method.

FIG. 10 shows the effect of different immobilized methods of 1-ethyl-3-methylimidazolium imidazole ionic liquid on the transesterification.

FIG. 11 is infrared spectrums of ionic liquid catalysts immobilized with different methods.

FIG. 12 is SEM image of heterogeneous catalyst of immobilized ionic liquid (Figure a shows a catalyst impregnated with 6g ionic liquid, Figures b, c and d show catalysts with 3g, 6g and 12g ionic liquid embedded, respectively).

FIG. 13 shows the physical photos of ethyl orthosilicate-magnesium acetate-zinc acetate-sodium aluminate-1-ethyl-3-methylimidazolium pyrrole salt under following states: during the hydrolysis, after the hydrolysis, after vacuum drying and after baking (A refers to during hydrolysis; B refers to after hydrolysis; C refers to after drying; and D refers to after baking).

FIG. 14 is XRD image of ethyl orthosilicate-magnesium acetate-zinc acetate-sodium aluminate-1-ethyl-3-methylimidazolium pyrrole salt after hydrolysis, drying and baking.

## DETAILED DESCRIPTION

The present disclosure will be described in detail below with examples, but is not limited to these examples.

Unless otherwise specified, the raw materials and catalysts in the examples of the present disclosure are commercially available.

In the examples of the present disclosure, 1HNMR analysis, 13CNMR analysis, infrared spectrum analysis, SEM analysis, and XRD analysis are all conventional operations, and those skilled in the art can operate according to the instructions of the instrument.

The conversion efficiency and selectivity in the examples of the present disclosure are calculated as follows.

In the examples of the present disclosure, the conversion efficiency and selectivity are calculated based on the molar number of carbon.

DMC conversion efficiency = (the molar number of produced EMC + the molar number of produced DEC) / (the molar number of unreacted DMC + the molar number of produced EMC + the molar number of produced DEC).

Ethanol (EtOH) conversion efficiency = (the molar number of produced EMC +2* the molar number of produced DEC)/( the molar number of unreacted EtOH + the molar number of produced EMC +2* the molar number of produced DEC).

EMC selectivity = (the molar number of produced EMC)/ (the molar number of produced EMC + the molar number of produced DEC).

DEC selectivity = (the molar number of produced DEC) / (the molar number of produced EMC + the molar number of produced DEC).

**Example 1**

The specific preparation method of the 1-ethyl-3-methylimidazolium imidazole salt was as follow: 0.5mol imidazole was dissolved in 60 mL ethanol solvent, and equimolar potassium ethoxide was added into a three-neck flask, and they were vigorously stirred for 6 h at 60°C; after the reaction was completed, rotary evaporation under reduced pressure was carried out at 65°C in an oil bath for 2h to remove the solvent ethanol and the product ethanol, and the remains were placed in a vacuum drying oven and dried for 12h until no weight change occurred to obtain the imidazolium potassium salt; 0.5mol commercially available [EmIm]Br was added into a three-neck flask, and under the condition that a water-carrying agent ethanol was used as solvent, equimolar imidazolium potassium was added therein, and they were stirred and reacted at room temperature for 24h; after the reaction was completed, the resulting white solid KBr was removed by filteration, rotary evaporation under reduced pressure was carried out at 65°C in an oil bath for 2h to remove the solvent, and then the remains were placed in a vacuum drying oven and dried for 12h until no weight change occurred, to obtain viscous liquid with light yellow, i.e., [EmIm]Im, of which nuclear magnetic resonance spectrums are shown in FIGs.1 and 2. 1HNMR spectrum of synthesized [EmIm]Im ionic liquid in FIG. 1 shows the followings: [EmIm]Im 1HNMR (500 MHz, DMSO-d6) δ (ppm): 8.61 (d, J = 1.9 Hz, 1H, NCHN), 7.75 (d, J = 1.9 Hz, 1H, NCH), 7.58 (s, 1H, NCH), 6.23 (s, 2H, NCHCHN), 4.17 (q, $J$ = 7.3 Hz, 2H, $NCH_2CH_3$), 3.83 (s, 3H, $NCH_3$), 1.37 (t, $J$ = 7.3 Hz, 3H, $NCH_2CH_3$). 13CNMR spectrum of synthesized [EmIm]Im ionic liquid in FIG. 2 shows the followings: $^{13}$C NMR (126 MHz, DMSO-$d_6$) δ (ppm): 166.43 (s), 136.99 (s), 136.18 (s), 123.97 (s), 122.39 (s), 122.31 (s), 44.51 (s), 36.06 (s), 15.56 (s). Therefore, the synthesized ionic liquid was determined to be 1-ethyl-3-methylimidazolium imidazole salt.

The specific preparation method of the 1-ethyl-3-methylimidazolium pyrrole salt is as follow: 0.5mol pyrrole was dissolved in 60 mL ethanol solvent, equimolar potassium ethoxide was added into a three-neck flask, and they were vigorously stirred for 6 h at 70°C; after the reaction was completed, rotary evaporation under reduced pressure was carried out for 2h at 65 °C in an oil bath to remove the ethanol solvent and the product ethanol, and the remains were placed into a vacuum drying oven and dried until no weight change occurred for 12h to obtain the pyrrole potassium; 0.5mol commercially available [EmIm]Br was added into a three-necked flask, and equimolar pyrrole potassium was added therein, and under the condition that a water-carrying agent ethanol is used as solvent, they were stirred at room temperature for 24h; after the reaction was completed, the resulting white solid KBr was removed by filtration, rotary evaporation under reduced pressure was carried out at 65°C in an oil bath for 2h to remove the solvent, and the remains were placed in a vacuum drying oven and dried for 12h until no weight change occurred to obtain viscous liquid with light yellow, i.e., [EmIm]py, of which nuclear magnetic resonance spectrums are shown in FIGs.3 and 4. 1HNMR spectrum of synthesized [EmIm]py ionic liquid in FIG. 3 shows the followings: [EmIm]Py$^1$H NMR (500 MHz, DMSO-$d_6$) δ (ppm): 9.30 (s, 1H, NCHN), 7.84 (s, 1H, NCHCHN), 7.74 (s, 1H, NCHCHN), 6.74 (d, $J$ = 1.9 Hz, 2H, NCHCH), 6.02 (d, $J$ = 1.5 Hz, 2H, CHCHCH), 4.20 (q, $J$ = 7.3 Hz, 2H,$NCH_2CH_3$), 3.85 (s, 3H, $NCH_3$), 1.39 (t, $J$=7.3 Hz, 3H, $NCH_2CH_3$). 13CNMR spectrum of synthesized [EmIm]py ionic liquid in FIG. 4 shows the followings: $^{13}$C NMR (126 MHz, DMSO-$d_6$) δ (ppm)

136.71 (s), 123.96 (s), 122.41 (s), 117.79 (s), 107.56 (s), 44.56 (s), 36.20 (s), 15.63 (s). Therefore, the synthesized ionic liquid was determined to be 1-ethyl-3-methylimidazolium pyrrole salt.

**Example 2**

Table 1 shows the comparisons of the activities of commercially available ionic liquids and ionic liquids synthesized in the laboratory for the transesterification of dimethyl carbonate and ethanol. Evaluation of the catalysts was carried out as follows: 46g ethanol and 90g dimethyl carbonate as reaction raw materials (a molar ratio thereof was 1:1) were evenly mixed in a 250 mL flask with magnetic stirring, the reaction system was heated to 76-78°C, about 2.72g ionic liquid was added therein after reflux was stable and the reaction time was 30-240 min, wherein the ionic liquid was 2% of the reaction raw material by weight. Wherein commercially available ionic liquids are as follows: 1-ethyl-3-methylimidazolium acetate, 1-ethyl-3-methylimidazolium chloride, 1-ethyl-3-methylimidazolium bromide, 1-ethyl-3-methylimidazolium iodide, 1-butyl-3-methylimidazolium fluoroborate, 1-butyl-3-methylimidazolium chloride, 1-butyl-3-methylimidazolium bromide, 1-butyl-3-methylimidazolium iodide, 1,3-dimethylimidazolium iodide. The ionic liquids synthesized in the laboratory are as follows: 1-ethyl-3-methylimidazolium methoxide, 1-ethyl-3-methylimidazolium hydroxide, 1-ethyl-3-methylimidazolium imidazole salt, 1-ethyl-3-methylimidazolium pyrrole salt, 1-ethyl-3-methylimidazolium morpholine salt, 1-butyl-3-methylimidazolium imidazole salt, 1,3-dimethylimidazolium pyrrole salt, N-methyl-N-ethylmorpholinium imidazole salt, N-methyl-N-ethylmorpholinium pyrrole salt, N-methyl-N-ethylmorpholinium morpholine salt, N-methyl-N-butylmorpholinium imidazole salt and N-methyl-N-butylmorpholinium pyrrole salt. The preparation method of the ionic liquid listed in Table 1 is the same as that of 1-ethyl-3-methylimidazolium imidazole salt in Example 2, except that the corresponding raw materials were changed.

Table 1

| The effect of different ionic liquids on the activity in the transesterification of DMC and EtOH | | | | | |
|---|---|---|---|---|---|
| Catalyst | Reaction time (min) | EtOH conversion efficiency (%) | DMC conversion efficiency (%) | EMC selectivity (%) | DEC selectivity (%) |
| 1-ethyl-3-methylimidazolium acetate | 180 | 25.37 | 20.43 | 88.88 | 11.12 |
| 1-ethyl-3-methylimidazolium chloride | 240 | 3.45 | 4.27 | 88.58 | 11.42 |
| 1-ethyl-3-methylimidazolium bromide | 120 | 16.46 | 15.35 | 89.99 | 10.01 |
| 1-ethyl-3-methylimidazolium iodide | 120 | 25.17 | 22.88 | 89.79 | 10.21 |
| 1-butyl-3-methylimidazolium fluoroborate | 180 | 0.08 | 0.11 | 79.87 | 20.13 |
| 1-butyl-3-methylimidazolium chloride | 120 | 1.52 | 1.52 | 89.95 | 10.05 |
| 1-butyl-3-methylimidazolium bromide | 120 | 8.83 | 8.17 | 89.56 | 10.44 |
| 1-butyl-3-methylimidazolium iodide | 120 | 13.38 | 12.57 | 88.25 | 11.75 |

(continued)

| The effect of different ionic liquids on the activity in the transesterification of DMC and EtOH | | | | | |
|---|---|---|---|---|---|
| Catalyst | Reaction time (min) | EtOH conversion efficiency (%) | DMC conversion efficiency (%) | EMC selectivity (%) | DEC selectivity (%) |
| 1,3-dimethylimidazolium iodide | 120 | 6.31 | 7.49 | 85.63 | 14.37 |
| 1-ethyl-3-methylimidazolium methoxide | 30 | 69.52 | 58.37 | 84.40 | 15.60 |
| 1-ethyl-3-methylimidazolium hydroxide | 30 | 69.68 | 59.22 | 80.93 | 19.07 |
| 1-ethyl-3-methylimidazolium imidazole salt | 30 | 69.62 | 61.45 | 75.72 | 24.28 |
| 1-ethyl-3-methylimidazolium pyrrole salt | 30 | 68.94 | 60.57 | 78.25 | 21.75 |
| 1-ethyl-3-methylimidazolium morpholine salt | 30 | 65.92 | 57.36 | 83.48 | 16.52 |
| 1-butyl-3 methylimidazolium imidazole salt | 30 | 67.83 | 60.32 | 78.03 | 21.97 |
| 1,3-dimethylimidazolium pyrrole salt | 30 | 70.02 | 62.42 | 77.36 | 22.64 |
| N-methyl-N-ethylmorpholinium imidazole salt | 30 | 70.36 | 62.15 | 77.23 | 22.77 |
| N-methyl-N-ethylmorpholinium pyrrole salt | 30 | 69.42 | 61.30 | 80.39 | 19.61 |
| N-methyl-N-ethylmorpholinium morpholine salt | 30 | 66.57 | 58.82 | 83.51 | 16.49 |
| N-methyl-N-butylmorpholinium imidazole salt | 30 | 65.68 | 58.75 | 84.33 | 15.67 |
| N-methyl-N-butylmorpholinium pyrrole salt | 30 | 65.03 | 57.83 | 85.18 | 14.82 |

The reaction conditions were as follows: a mole ratio of DMC to EtOH in the raw material was 1:1, the amount of the catalyst was 2 wt%, and the reaction temperature was in a range from 76°C to 78°C.

As seen from the data in Table 1, the commercially available imidazole ionic liquids have low activity, and the conversion efficiency of dimethyl carbonate was less than 25% when the reaction was carried out for 120 minutes which cannot meet the conditions of the homogeneous catalytic synthesis process of ethyl methyl carbonate (reaction temperature of 64-80°C, reaction equilibrium time of around 30 min). In contrast, under the condition that imidazole-based ionic liquids with various structures synthesized in the laboratory were used and the reaction was carried out for 30 min, the conversion efficiency of dimethyl carbonate was close to 60% and the conversion efficiency of ethanol was close to 70% which are close to the equilibrium conversion efficiency at this temperature, and the selectivity of ethyl methyl carbonate was about 80%, indicating that the new imidazole ionic liquids synthesized in the laboratory showed higher catalytic activity in the transesterification of dimethyl carbonate and ethanol ester.

**Example 3**

1-butyl-3-methylimidazolium pyrrole salt was used as catalyst of which the initial state was shown in FIG. 5, and the color of the prepared ionic liquid was transparent light yellow.

The reuse evaluation of 1-butyl-3-methylimidazolium pyrrole salt catalyst was carried out as follows: 46g ethanol and 90g dimethyl carbonate as reaction raw materials (a molar ratio thereof was 1:1) were evenly mixed in a 250 mL flask with magnetic stirring, the reacting system was heated to 76-78°C, about 2.72g 1-butyl-3-methylimidazolium pyrrole salt ionic liquid ( i.e., the ionic liquid was 2% of the reaction raw materials by weight) was added therein after the reflux was stable, the reaction time was 30 min; after the reaction was completed, the reaction raw materials, products and ionic liquid were subject to rotary evaporation under reduced pressure at 110 °C for 30 min, and the remaining liquid in the flask for rotary evaporation was 1-butyl-3-methylimidazolium pyrrole salt catalyst which was directly used for the next reaction evaluation. FIG. 6 showed remaining ionic liquid in the flask for rotary evaporation after the ionic liquid was reused 20 times, and the color thereof turned slightly yellow compared with the color of the initial ionic liquid. FIG.7 showed how the conversion efficiency of dimethyl carbonate and ethanol, and the selectivity of ethyl methyl carbonate and diethyl carbonate varied with reused times. The conversion efficiency of dimethyl carbonate was basically maintained at about 60%, the conversion efficiency of ethanol was basically maintained at about 70%, the selectivity of ethyl methyl carbonate was maintained at around 80%, and the selectivity of diethyl carbonate was 20%, indicating that the synthesized 1-butyl-3-methylimidazolium pyrrole salt catalyst had good stability and basically unchanged activity after reused 20 times.

**Example 4**

1-ethyl-3-methylimidazolium imidazole salt was used as catalyst, and the effects of rust and iron ion on the activity and reused stability of the synthesized ionic liquid in industrial applications were investigated. A mixture of ferric oxide and ferroferric oxide was used to simulate the rust component, and ferric chloride was used as the source of iron ion and chloride ion. Evaluation of the catalysts was carried out as follows: 46g ethanol and 90g dimethyl carbonate as reaction raw materials (a molar ratio thereof was 1:1) were evenly mixed with a magnetic stirring in a 250 mL flask, the reacting system was heated to a range from 76°C to 78°C, about 1.36g 1-ethyl-3-methylimidazolium imidazole salt ionic liquid (i.e., the ionic liquid was 1% of the reaction raw materials by weight) was added therein after reflux was stable, the reaction time was 30 min, 0.1wt% ferric oxide and 0.1wt% ferroferric oxide of the catalyst were added therein respectively while 0.1wt% ferric chloride of the catalyst was added in another experiment; after the reaction was completed, the reaction raw materials, products, ionic liquid, and ferric oxide, ferroferric oxide or ferric chloride were subject to rotary evaporation under reduced pressure at 110°C for 30 min, and the remains (comprising liquid and solid) in the flask for rotary evaporation were directly used for the next reaction evaluation. Table 2 showed how the activity varied with reused times when the rust existed in the ionic liquid.

Table 2

| Effects of ferric oxide and ferroferric oxide on the catalytic activity of the reused ionic liquids | | | | |
|---|---|---|---|---|
| Reused times | EtOH conversion efficiency (%) | DMC conversion efficiency (%) | EMC selectivity (%) | DEC selectivity (%) |
| initial | 61.33 | 58.36 | 81.99 | 18.01 |
| 1 | 61.06 | 56.43 | 83.68 | 16.32 |
| 2 | 57.68 | 52.01 | 83.63 | 16.37 |
| 3 | 56.97 | 53.07 | 84.16 | 15.84 |
| 4 | 56.78 | 52.05 | 84.22 | 15.78 |
| 5 | 58.95 | 55.71 | 84.90 | 15.10 |
| 6 | 57.42 | 54.38 | 86.73 | 13.27 |

The reaction conditions were as follows: a mole ratio of DMC to EtOH in the raw material was 1:1, the catalyst was 1wt% of the reaction solution, 0.1wt% ferric oxide and 0.1wt% ferroferric oxide were added and the reaction was carried out at a temperature ranging from 76°C to 78°C. Note: Ferric oxide and ferroferric oxide were insoluble in the solution during the reaction.

Under the condition that ferric oxide and ferroferric oxide were in the reaction system and the reaction was carried out for 30 min, the conversion efficiency of dimethyl carbonate and ethanol was slightly reduced compared with the initial use of the catalyst, significant activity change did not occur during reuse and basically remained stable, but the selectivity of ethyl methyl carbonate had an increasing trend.

Table 3

| Effects of ferric chloride on the activity of reused 1-ethyl-3-methylimidazolium imidazole salt | | | | |
|---|---|---|---|---|
| Reused times | EtOH conversion efficiency (%) | DMC conversion efficiency (%) | EMC selectivity (%) | DEC selectivity (%) |
| initial | 58.46 | 55.83 | 84.66 | 15.34 |
| 1 | 9.42 | 6.43 | 79.70 | 20.30 |
| 2 | 1.73 | 2.85 | 94.62 | 5.38 |
| 3 | 1.13 | 1.11 | 97.69 | 2.31 |

Note: the initial catalyst was 1wt% of the reaction solution, and the reaction temperature was in a range from 76 °C to 78°C under normal pressure for 30 min. Reuse of 0 time without ferric chloride was used as reference experiment. Ferric chloride was added for reuse of 1-3 time(s) therein, and the resulting sample became reddish brown. Except that the reuses of 0 and 1 time resulted in colorless and clear filtrate, all the rest filtrate showed lemon color (that is, pale golden yellow).

Under the condition that ferric chloride was in the reaction system, compared with initial 1-ethyl-3-methylimidazolium imidazole salt in Table 1, the conversion efficiency of dimethyl carbonate decreased by about 4%, and the conversion efficiency of ethanol decreased by about 11%, indicating that the addition of ferric chloride significantly reduced the activity of the prepared ionic liquid. When the ionic liquid was reused, the catalytic activity was significantly reduced. When the ionic liquid was reused 2 times, the conversion efficiency of dimethyl carbonate and ethanol was already below 3%. Thus, it should be noted that iron ion should be avoided for the prepared ionic liquids in the industrialization for preparing ethyl methyl carbonate and diethyl carbonate by transesterification of dimethyl carbonate and ethanol.

**Example 5**

0.136g 1,3-dimethylimidazolium pyrrole was used as catalyst, 46g ethanol and 90g dimethyl carbonate reaction raw material (a molar ratio thereof was 1:1) were mixed in a 250mL flask with a magnetic stirring, the reaction was carried out at 30°C for 1min, 3min, 5min, 7min, 10min, 15min, 20min, 30min, 45min, 60min and 90 min respectively, and the results were shown in Table 4. 0.2wt% 1,3-dimethylimidazolium pyrrole was used as catalyst, and the reaction was carried out at 50°C for 1min, 2min, 3min, 4min, 5min, 6min, 7min, 8min, 10min, 20min, and 30min respectively, and the results were shown in Table 5.

Table 4

| Variations of conversion efficiency of the raw materials and selectivity of the products with the reaction time (0.1%, 30°C) | | | | |
|---|---|---|---|---|
| Time/min | EtOH conversion efficiency (%) | DMC conversion efficiency (%) | EMC selectivity (%) | DEC selectivity (%) |
| 1 | 18.65 | 17.38 | 93.89 | 6.11 |
| 3 | 24.80 | 23.02 | 96.71 | 3.29 |
| 5 | 31.10 | 28.99 | 94.00 | 6.00 |
| 7 | 34.21 | 31.17 | 95.53 | 4.47 |
| 10 | 36.36 | 34.19 | 95.04 | 4.96 |

(continued)

| Variations of conversion efficiency of the raw materials and selectivity of the products with the reaction time (0.1%, 30°C) | | | | |
|---|---|---|---|---|
| Time/min | EtOH conversion efficiency (%) | DMC conversion efficiency (%) | EMC selectivity (%) | DEC selectivity (%) |
| 15 | 41.03 | 37.88 | 94.10 | 5.90 |
| 20 | 43.23 | 39.16 | 93.91 | 6.09 |
| 30 | 46.74 | 42.31 | 93.02 | 6.98 |
| 45 | 48.36 | 44.20 | 92.39 | 7.61 |
| 60 | 49.56 | 45.29 | 92.15 | 7.85 |
| 90 | 52.17 | 46.56 | 91.64 | 8.36 |

Under the condition that 1,3-dimethylimidazolium pyrrole was used as catalyst, the content thereof was only 0.1% and the reaction was carried out at only 30°C, the conversion efficiency of dimethyl carbonate and ethanol gradually increased with the reaction time (1-90 min), the selectivity of ethyl methyl carbonate was kept above 90%, and the selectivity of diethyl carbonate was below 10%, indicating that the prepared ionic liquid also had better activity under low temperature conditions.

Under the condition that the reaction temperature was raised to 50°C and the content of the 1,3-dimethylimidazole pyrrole catalyst was 0.2%, the catalytic activity was significantly improved. When the reaction was carried out for only 1 min, the conversion efficiency of dimethyl carbonate reached 38.35% and the ethanol conversion efficiency reached 40.18%. When the reaction time was extended to 5 min, the conversion efficiency of dimethyl carbonate reached above 50% and the conversion efficiency of ethanol was close to 60%. The selectivity of ethyl methyl carbonate basically followed the gradually decreasing trend with the reaction time.

Table 5

| Variations of conversion efficiency of the raw material and selectivity of the products with the reaction time (0.2%, 50°C) | | | | |
|---|---|---|---|---|
| Time/min | EtOH conversion efficiency (%) | DMC conversion efficiency (%) | EMC Selectivity (%) | DEC Selectivity (%) |
| 1 | 40.18 | 38.35 | 94.27 | 5.73 |
| 2 | 45.56 | 42.75 | 93.16 | 6.84 |
| 3 | 51.01 | 46.58 | 93.67 | 3.45 |
| 4 | 53.95 | 49.50 | 90.76 | 9.24 |
| 5 | 58.94 | 51.60 | 86.81 | 13.19 |
| 6 | 59.21 | 52.20 | 87.60 | 12.41 |
| 7 | 59.75 | 53.56 | 88.55 | 11.45 |
| 8 | 62.68 | 55.86 | 87.81 | 12.19 |
| 10 | 63.60 | 56.70 | 87.61 | 12.39 |
| 20 | 66.74 | 58.94 | 85.13 | 14.87 |
| 30 | 66.11 | 57.39 | 85.07 | 14.93 |

**Example 6**

All types of prepared new ionic liquids could also be applied to the transesterification of dimethyl carbonate and diethyl carbonate to generate ethyl methyl carbonate, and the activities of the prepared ionic liquids with strong alkalinity and having different structures were not much different. Taking 1-ethyl-3 methylimidazolium imidazole salt as an example, 45g dimethyl carbonate and 59g diethyl carbonate were evenly mixed in a 250mL three-neck flask, and 0.52g (i.e., 0.5% of the total weight of the reaction raw materials) catalyst was added therein immediately when the

reaction temperature reached 92-94°C. Table 6 showed the results of chromatographic analysis when the reaction was carried out for 5 min, 15 min, 30 min, 60 min, 120 min, and 240 min, respectively.

Table 6

| Results of the transesterification of DMC and DEC catalyzed by 1-ethyl-3 methylimidazolium imidazole salt | | | | | |
|---|---|---|---|---|---|
| Reaction time /min | Conversion efficiency /% | | Weight distribution/% | | |
| | DMC | DEC | DMC | EMC | DEC |
| 5 | 8.19 | 8.96 | 41.75 | 8.61 | 49.64 |
| 15 | 33.23 | 33.53 | 29.04 | 33.40 | 37.56 |
| 30 | 50.41 | 51.22 | 21.65 | 50.86 | 27.49 |
| 60 | 53.18 | 54.73 | 20.58 | 54.05 | 25.37 |
| 120 | 55.33 | 55.26 | 19.31 | 55.29 | 25.40 |
| 240 | 54.07 | 55.36 | 20.14 | 54.79 | 25.07 |

The reaction conditions were as follows: a mole ratio of DMC to DEC was 1:1 (DMC 45g, DEC 59g), content of ionic liquid catalyst was 0.5wt%, and the reaction was carried out at 92-94°C in a flask.

Since the molar ratio of DMC and DEC in the reaction was 1:1 and no side reaction occurred, the conversion efficiency of DMC and DEC were approximately the same in different reaction time periods. As shown in Table 6, the conversion efficiency of DMC and DEC gradually increased with the reaction time. When the reaction time was more than 30 min, the conversion efficiency of DMC and DEC reached above 50%. When the reaction time was more than 60 min, the conversion efficiency of DMC and DEC were maintained at about 55%, basically reaching reaction equilibrium. DMC was about 20% of the equilibrium components by weight, EMC was about 54% of the equilibrium components by weight, and DEC was about 25% of the equilibrium components by weight. A reaction equilibrium could be basically reached when the reaction of DMC and DEC catalyzed by 0.5wt% 1-ethyl-3-methylimidazolium imidazole salt for 30 min or more, indicating that the prepared ionic liquid also had the ability to efficiently catalyze the transesterification of DMC and DEC.

**Example 7**

All types of prepared new ionic liquids could also be applied to the reaction of methanol and diethyl carbonate to generate ethyl methyl carbonate and ethanol, and the activities of the prepared ionic liquids with strong alkalinity and having different structures were not much different. Taking 1-ethyl-3-methylimidazolium pyrrole salt as an example, 19.2g methanol and 70.8g diethyl carbonate were evenly mixed in a 250 mL three-neck flask, 0.45g catalyst (i.e., 0.5% of the total weight of the reaction raw materials) was added therein immediately when the reaction temperature reached 70-72°C. Table 7 showed the results of chromatographic analysis when the reaction was carried out for 5 min, 15 min, 30 min, 60 min, 120 min, and 240 min, respectively.

Table 7

| Results of the transesterification of DEC and MeOH catalyzed by 1-ethyl-3 methylimidazolium pyrrole salt | | | | |
|---|---|---|---|---|
| Reaction time /min | MeOH conversion efficiency (%) | DEC conversion efficiency (%) | EMC selectivity (%) | DMC selectivity (%) |
| 5 | 34.87 | 33.41 | 86.17 | 13.83 |
| 15 | 39.79 | 28.63 | 88.07 | 11.93 |
| 30 | 40.58 | 33.33 | 86.76 | 13.24 |
| 60 | 50.30 | 37.34 | 85.55 | 14.45 |
| 120 | 55.39 | 46.92 | 82.68 | 17.32 |
| 180 | 64.40 | 52.80 | 82.18 | 17.82 |
| 240 | 71.16 | 56.05 | 83.34 | 16.66 |
| 360 | 67.62 | 58.66 | 83.18 | 16.82 |

The reaction conditions were as follows: a mole ratio of MeOH to DEC was 1:1(MeOH 19.2g, DEC 70.8g), content of ionic liquid catalyst was 0.5wt%, and the reaction was carried out at 70-72°Cin a three-neck flask.

The reaction of methanol and DEC was stepwise. In the first step, methanol and DEC were reacted to generate EMC and ethanol, while in the second step, methanol and EMC were reacted to generate DMC and ethanol. No other side reaction occurred. Table 7 showed the conversion efficiency of methanol and DEC, and the selectivity of EMC and DMC. The conversion efficiency of methanol and DEC gradually increased with the reaction time. Due to the higher proportion of methanol in the reaction system, the reaction temperature thereof was lower than that of the transesterification of DMC and ethanol or that of the transesterification of DMC and DEC, resulting in slightly lower reaction efficiency. When the reaction was carried out for 240 min, a reaction equilibrium was basically reached, and the EMC selectivity was about 83%.

**Example 8**

Under a single silica obtained by hydrolyzing ethyl orthosilicate as support, the following described in detail acid-catalyzed hydrolysis of ethyl orthosilicate, alkali-catalyzed hydrolysis of ethyl orthosilicate, conventional impregnation of silica support with ionic liquid, and preparation method of heterogeneous catalyst with ionic liquid embedded in silica in a riveting manner.

**Preparation of silica support:** 30mL ethyl orthosilicate and 21mL ethanol were respectively added into a conical flask and stirred at room temperature for 10 min, and then 9mL 0.45mol/L NaOH or 0.5mol/mL hydrochloric acid solution was added therein immediately when they were heated to 60 °C. Ethyl silicate slowly was hydrolyzed, and the system gradually gelled. After the gelled system was aged at 60°C for 12h, the resulting mixture was washed three times with 50 mL ethanol, and vacuum dried at 150°C for 3h to obtain silica support by alkali/acid-promoted hydrolysis.

**Impregnating method with ionic liquid:** 8g pure support was placed in a watch glass, then placed in an ultrasonic machine to carry out ultrasonic operation, and at room temperature, 12g 1-ethyl-3 methylimidazolium imidazole ionic liquid was dropped slowly to the support within 30 min with continuous stirring; after the dropping was completed, stirring was maintained for 30 min. Then the support was taken out and dried at 120°C for 10 h, then washed three times with 50 mL ethanol, and vacuum-dried at 150°C for 3 h to obtain the impregnated ionic liquid.

**Method of embedding ionic liquid in a riveting manner:** 30mL ethyl orthosilicate and 21mL ethanol were added into an conical flask respectively, and when they were heated to 60°C, 3g 1-ethyl-3methylimidazolium imidazole ionic liquid, 6g 1-ethyl-3methylimidazolium imidazole ionic liquid or 12g 1-ethyl-3methylimidazolium imidazole ionic liquid were immediately added therein, and after even mixing thereof, 8.9g deionized water was added therein. Among them, 1-ethyl-3 methylimidazolium imidazole ionic liquid serves both as alkali catalyst to promote the hydrolysis of ethyl orthosilicate and as active center of carbonate transesterification. After deionized water was added therein, the system gelled quickly. The gelled system was aged at 60°C for 12h, dried at 120°C for 10h, then washed with 50 mL ethanol three times, and vacuum dried at 150°C for 3h to obtain a heterogeneous catalyst with 3g, 6g or 12g ionic liquid immobilized.

FIGS. 8 and 9 showed the physical photos of the catalysts immobilized by impregnating method with ionic liquid and prepared by embedding Ionic liquid in a riveting manner, respectively.

As can be seen from the physical photos, whether the catalyst was prepared by impregnating method with ionic liquid or prepared by embedding ionic liquid in a riveting manner, the apparent color of the catalyst was uniform light yellow and the texture thereof was crunchy. The appearance of the catalyst prepared by these two methods was not much different.

**Example 9**

A fixed-bed reactor was used to evaluate the prepared heterogeneous catalyst. Reaction conditions were as follows: a molar ratio of DMC to EtOH as reaction raw materials was 1: 1, the reaction temperature was in a range from 76°C to 78°C, filling weight of the catalyst was 4 g, weight hourly space velocity of the reaction raw materials was $1h^{-1}$, that is, feeding weight per hour for the raw materials was 4g, and the continuous evaluation time for each catalyst was 2300 min. Table 8 showed the catalyst activity results of chromatographic analysis at the stage of stabilization. The heterogeneous catalysts listed in Table 8 were as follows: silica support obtained by alkali-catalyzed hydrolysis, silica support obtained by acid-catalyzed hydrolysis, silica impregnated with 12g imidazolium potassium (the preparation method thereof was the same as the impregnating method with ionic liquid except that 12g imidazolium potassium was used), silica with 12g imidazolium potassium embedded (the preparation method thereof was the same as the method for embedding ionic liquid in a riveting manner except that 12g imidazolium potassium was used), silica impregnated with 12g imidazolium pyrrole (the preparation method thereof was the same as the impregnating method with ionic liquid except that 12g imidazolium pyrrole was used), silica with 12g imidazolium pyrrole embedded (the preparation method thereof was the same as the method for embedding ionic liquid in a riveting manner except that 12g imidazolium pyrrole was used), silica impregnated with 12g morpholinium imidazole (the preparation method thereof was the same as the

impregnating method with ionic liquid except that 12g morpholinium imidazole was used), and silica with 12g morpholinium imidazole embedded (the preparation method thereof was the same as the method for embedding ionic liquid in a riveting manner except that 12g morpholinium imidazole was used), silica impregnated with 12g morpholinium pyrrole (the preparation method thereof was the same as the impregnating method with ionic liquid except that 12g morpholinium pyrrole was used) and silica with 12g morpholinium pyrrole embedded (the preparation method thereof was the same as the method for embedding ionic liquid in a riveting manner except that 12g morpholinium pyrrole was used).

As shown in Table 8, the catalytic activity of both the silica support obtained by acid-catalyzed hydrolysis and the silica support obtained by alkali-catalyzed hydrolysis was less than 5%, indicating that the silica support alone had little ability to catalyze the transesterification of DMC and EtOH. The catalytic activity of the silica impregnated with imidazolium potassium was also less than 5%,which was similar to that of the silica support, indicating that imidazolium potassium impregnated in the pores of the silica could be easily washed away with ethanol. The catalytic activity of silica with potassium imidazole embedded could be maintained at 20.99% of DMC conversion efficiency and 23.24% of EtOH conversion efficiency, indicating that imidazolium potassium, even having single ring structure, could be relatively stably embedded in the grid structure of silica. If a double-ring structure was embedded in the silica framework, the conversion efficiency of DMC and EtOH as raw materials would be significantly higher than that achieved by imidazolium potassium, and was maintained at 30%-35%. However, the impregnated double-ring structure showed an obvious phenomenon of being washed away by ethanol, and the conversion efficiency of DMC and EtOH ranged between 5%-10%. In summary, compared with the catalyst prepared by impregnating method, the catalyst prepared by embedding method exhibited superior catalytic activity and stability, indicating that in comparison with the conventional impregnating method, the developed immobilized method of ionic liquid was a more reliable immobilized method of active component.

Table 8

| Effect of ionic liquid catalyst immobilized by different methods on the reaction of DMC and EtOH | | | | |
|---|---|---|---|---|
| Catalyst | DMC conversion efficiency (%) | Ethanol conversion efficiency (%) | EMC selectivity (%) | DEC selectivity (%) |
| $SiO_2$ support obtained by alkali-catalyzed hydrolysis | 3.15 | 4.20 | 96.08 | 3.92 |
| $SiO_2$ support obtained by acid-catalyzed hydrolysis | 1.28 | 2.24 | 96.98 | 3.02 |
| $SiO_2$ impregnated with imidazolium potassium | 2.43 | 3.35 | 96.64 | 3.36 |
| $SiO_2$ with imidazolium potassium embedded | 20.99 | 23.24 | 94.49 | 5.51 |
| $SiO_2$ impregnated with imidazolium pyrrole | 8.72 | 10.09 | 90.63 | 9.37 |
| $SiO_2$ with imidazolium pyrrole embedded | 34.23 | 35.85 | 88.73 | 11.27 |
| $SiO_2$ impregnated with morpholinium imidazole | 8.24 | 9.61 | 90.77 | 9.23 |
| $SiO_2$ with morpholinium imidazole embedded | 29.78 | 35.45 | 89.29 | 10.71 |
| $SiO_2$ impregnated with morpholinium pyrrole | 5.53 | 5.72 | 97.67 | 2.33 |
| $SiO_2$ with morpholinium pyrrole embedded | 29.77 | 36.37 | 82.85 | 17.15 |
| DMC/EtOH=1/1, embedding weight was 6g, reaction temperature was in a range from 76°C to 78 °C, the filling weight of the catalyst was 4g, weight hourly space velocity was $1h^{-1}$, and reaction time was 2300min | | | | |

**Example 10**

A fixed-bed reactor was used to evaluate the following catalysts: 12g 1-ethyl-3-methylimidazolium imidazole ionic liquid @silica prepared by impregnating method (the preparation method thereof was the same as was the same as the impregnating method with ionic liquid except that 1-ethyl-3-methylimidazolium imidazole ionic liquid was used) and 1-ethyl-3-methylimidazolium imidazole ionic liquid @silica prepared by embedding method (the preparation method thereof was the same as the method for embedding ionic liquid in a riveting manner except that 1-ethyl-3-methylimidazolium imidazole ionic liquid was used). A molar ratio of DMC to EtOH as reaction raw materials was 1: 1, the reaction temperature was in a range from 76°C to 78°C, the filling weight of the catalyst was 4g, and weight hourly space velocity of the reaction raw materials was 1 $h^{-1}$. FIG. 10 showed how the conversion efficiency of the DMC and EtOH as raw materials and EMC selectivity varied with time.

Under the heterogeneous catalyst prepared by impregnating method was used, the conversion efficiency of both DMC and EtOH at initial stage of the reaction was close to 20% and the heterogeneous catalyst was deactivated seriously with time, the conversion efficiency of both DMC and EtOH decreased to about 15% when the reaction was carried out for 1000 min, and the conversion efficiency of both DMC and EtOH decreased to below 10% while EMC selectivity increased from 85% to about 95% when the reaction was carried out for 2000min. Under 1-ethyl-3-methylimidazolium imidazole ionic liquid@silica catalyst prepared by embedding method was used, the conversion efficiency of both DMC and EtOH reached above 60% at the initial stage of the reaction, and the catalyst activity was not decreased with time, and even the conversion efficiency of ethanol increased slightly, indicating that the catalyst prepared by embedding method had good stability.

**Example 11**

The reaction activity of the catalyst immobilized by embedding method and selectivity of the product were also affected by the hydrolysis temperature, aging time, aging temperature and embedding weight of active component. Table 9 showed how conversion efficiency of DMC and ethanol and product selectivity varied with different catalyst preparation conditions. The specific preparation method was the same as the method for embedding ionic liquid in a riveting manner in Example 8 except for the aging time, aging temperature, and the content of the active component. Under the condition that the hydrolysis temperature was 60°C, the aging temperature was 60°C, the aging time was 12h and the embedding weight was 12g, the prepared catalyst had the best activity.

Table 9

| Effect of different embedding conditions on the carbonate transesterification | | | | | |
|---|---|---|---|---|---|
| Factors | Variable Conditions | DMC conversion efficiency (%) | Ethanol conversion efficiency (%) | EMC selectivity (%) | DEC selectivity (%) |
| Hydrolysis temperature | 50°C | 32.74 | 33.54 | 88.49 | 11.51 |
| | 60°C | 62.52 | 62.59 | 75.71 | 24.29 |
| | 70°C | 33.94 | 32.62 | 90.18 | 9.82 |
| Aging temperature | 50°C<br>60 °C | 25.11<br>62.52 | 27.45<br>62.59 | 86.43<br>75.71 | 13.57<br>24.29 |
| | 70°C | 34.23 | 35.85 | 88.73 | 11.27 |
| Aging time | 5h<br>12 h | 7.10<br>62.52 | 7.91<br>62.59 | 80.42<br>75.71 | 19.58<br>24.29 |
| | 20 h | 57.29 | 69.04 | 72.13 | 27.87 |
| Embedding weight of active component | 3 g | 5.51 | 5.71 | 96.99 | 3.01 |
| | 6 g<br>12 g | 29.77<br>62.52 | 36.37<br>62.59 | 82.85<br>75.71 | 17.15<br>24.29 |

A molar ratio of DMC to EtOH as raw materials was 1:1, the ionic liquid was 1-ethyl-3-methylimidazolium imidazole, a reaction temperature was 76-78°C, filling weight of the catalyst in the fixed-bed was 4g, weight hourly space velocity was 1$h^{-1}$, and reaction time was 2300min

### Example 12

Figure 11 showed infrared spectrums of the catalysts obtained by impregnating silica as support with 6g 1-ethyl-3-methyl-imidazolium imidazole salt and the catalysts obtained by embedding 3g, 6g and 12g 1-ethyl-3-methyl-imidazolium imidazole salt in silica as support, respectively. The strong absorption peak at 1064$cm^{-1}$ was the stretching vibration characteristic peak of Si-O-Si, the absorption peak at 3427$cm^{-1}$ was the stretching vibration peak of alcoholic O-H group, the absorption peak at 1631$cm^{-1}$ was the characteristic absorption peak of C=C on the imidazole ring, and the characteristic absorption peak of the imidazole ring was at 797$cm^{-1}$. It showed that the developed method for preparing immobilized ionic liquid catalyst in an embedding manner was reliable and feasible, and the active component ionic liquid was determined to be embedded in the heterogeneous catalyst. The height of the characteristic peak of imidazole in the prepared catalyst increased strictly and regularly in accordance with the law of embedding weight of 3, 6 and 12g. The catalyst prepared by impregnating with 6g ionic liquid also showed that characteristic peak intensity of imidazole thereof was higher than that shown by the catalysts obtained by embedding 3g ionic liquid, but lower than that shown by the catalysts obtained by embedding 6g ionic liquid, which was substantially consistent with their catalytic activity.

### Example 13

Figure 12 showed SEM images of the catalysts obtained by impregnating silica as support with 6g 1-ethyl-3-methyl-imidazolium imidazole salt and the catalysts obtained by embedding 3g, 6g or 12g 1-ethyl-3-methyl-imidazolium imidazole salt in silica as support, respectively.

As shown in Figure 12, Figure a shows an apparent morphology of a strip structure while Figure b shows a regular morphology of silica molecules, the reason of which is that the amount of ionic liquid added is not enough to form a messy composite structure of silane support and ionic liquid. Figures c and d show great changes of apparent morphology of the embedded ionic liquid, and the messy small particles accumulated on the support to form a random composite of silane support and ionic liquid, further illustrating that the ionic liquid was successfully loaded on the support via silane coupling and the cluster phenomenon was more obvious as the loading weight increased. These SEM images fully illustrated that the synthesized ionic liquids by embedding method were distributed in the framework of silica via silane coupling, and the apparent microstructure thereof was significant different from the apparent microstructure of silica.

### Example 14

N-methyl-N-butylmorpholinium imidazole salt ionic liquid was embeded in support of tetrabutyl titanate and support of the combination of ethyl orthosilicate and tetrabutyl tantalate respectively. A: 30 mL tetrabutyl titanate and 21 mL ethanol were added into a conical flask, and when they were heated to 60°C, 8g N-methyl-N-butylmorpholinium imidazole salt ionic liquid was added therein immediately, and after even mixing, 8.9g deionized water was added therein, then the resulting mixture was aged at 60°C for 12h, dried at 120°C for 10h, then washed three times with 50mL ethanol, and vacuum dried at 150°C for 3h to obtain immobilized catalyst of the ionic liquid. B: 14.5mL ethyl orthosilicate, 23 mL tetrabutyl titanate and 60mL ethanol were added into a conical flask respectively, and when they were heated to 60°C, 10g N-methyl-N-butylmorpholinium imidazole salt ionic liquid was added therein, and 2.0g calcium acetate, 3.8g lanthanum acetate, 3.6g iron acetate, 1.8g lithium acetate, and 2.5g sodium aluminate were further added therein, and after uniformly stirring, 12.34g deionized water was added therein; the resulting mixture was aged at 60°C for 12h, dried at 120°C for 10h, then washed three times with 60mL ethanol, and vacuum dried at 150°C for 3h to obtain immobilized catalyst of the ionic liquid.

Table 10

| Results of three kinds of carbonate transesterifications catalyzed by ionic liquid embedded in the tetrabutyl titanate-based support | | | | | |
|---|---|---|---|---|---|
| Catalyst | Reaction raw material and Reaction temperature (°C) | Raw material 1 conversion efficiency (%) | Raw material 2 conversion efficiency (%) | Product 1 selectivity (%) | Product 2 selectivity (%) |
| A | EtOH/DMC=1/1 (76-78) | 58.39 | 53.35 | 82.83 | 17.17 |
| | DMC/DEC=1/1 (90-92) | 43.16 | 43.42 | 100 | - |
| | MeOH/DEC=1/1 (70-72) | 41.24 | 37.98 | 86.76 | 13.24 |
| B | EtOH/DMC=1/1 (76-78) | 63.17 | 58.46 | 80.30 | 19.70 |
| | DMC/DEC=1/1 (90-92) | 48.86 | 49.01 | 100 | - |
| | MeOH/DEC =1/1 (70-72) | 46.25 | 40.17 | 85.55 | 14.45 |

A filling weight of catalyst in the fixed bed was 4g, weight hourly space velocity was $1h^{-1}$, and reaction time was 1000min.

Reaction raw material 1 referred to the reactant on the left of notation'/', the raw material 2 referred to the reactant on the right of notation '/', the product 1 referred to EMC, and the product 2 referred to DEC or DMC.

As shown in Table 10, when the weight hourly space velocity of the reaction raw materials was $0.2h^{-1}$, the heterogeneous catalysts prepared by embedding method, in which $TiO_2$ obtained by hydrolyzing tetrabutyl titanate, or hydrolysate obtained by hydrolyzing the combination of ethyl orthosilicate and tetrabutyl titanate was used as support, and N-methyl- N-butylmorpholinium imidazole salt ionic liquid was used as active component, exhibited high reactivity for transesterification of dimethyl carbonate and ethanol, transesterification of dimethyl carbonate and diethyl carbonate, or transesterification of methanol and diethyl carbonate, and the products were only ethyl methyl carbonate, diethyl carbonate, methanol or ethanol, and no other products were detected at all. The catalyst obtained by embedding N-methyl-N-butylmorpholinium imidazole salt ionic liquid in a combination of ethyl orthosilicate, tetrabutyl titanate and a variety of metal cations, had slightly higher activity than the catalyst obtained by embedding N-methyl-N-butylmorpholinium imidazole salt ionic liquid in single support of tetrabutyl titanate. It was speculated that when $Li^+$, $Al^{3+}$, $La^{3+}$, $Fe^{3+}$, and $Ca^{2+}$ entered the framework of silica and titania, they could stabilize the structure thereof, increase the embedding amount of ionic liquid, and thus made the catalyst B exhibit better reactivity than the catalyst A for all the three reactions.

**Example 15**

Ethyl orthosilicate was used support to embed 1-ethyl-3-methylimidazolium pyrrole salt ionic liquid to prepare the catalyst. 31.5mL ethyl orthosilicate and 50mL ethanol were added into a conical flask, and when they were heated to 60°C, 20g 1-ethyl-3-methylimidazolium pyrrole salt ionic liquid was added therein, 8.73g magnesium acetate, 5.5g zinc acetate, 4.06g aluminum acetate were further added therein and after even mixing, 9.35g deionized water was added therein, the resulting mixture was aged at 60°C for 12 h, dried at 120°C for 10 h, then washed three times with 50mL ethanol, and vacuum dried at 150°C for 3h to obtain immobilized catalyst of the ionic liquid. FIG. 13 showed photos of catalyst precursors during the hydrolysis, after the hydrolysis, after drying and after baking. As shown in FIG. 13A, during the hydrolysis, uniform brown-yellow solution was formed, and ethyl orthosilicate, 1-ethyl-3-methylimidazolium pyrrole salt ionic liquid and various acetates could be completely dissolved in the ethanol solvent. As shown in FIG. 13B, after hydrolysis, the precursor formed completely uniform brown-yellow solid, and there was basically no liquid phase remaining in the system. As shown in FIG.13C, the gelled mixture after drying had significantly lighter color than the colloid after hydrolysis, and was crunchy powder with light brown yellow. As shown in FIG. 13D which showed photo of colloid C after baking at 550°C for 3 h in an air atmosphere, a brown powdery product was obtained. We inferred that the reason why the solid product appeared brown was that 1-ethyl-3-methylimidazolium pyrrole ionic liquid embedded in the bulk phase (which was distributed in frameworks of silica and $Mg^{2+}$, $Zn^{2+}$, and $Al^{3+}$) decomposed incompletely by heat, thereby resulting in a large amount of remaining carbon deposits. It just right indirectly proved that the ionic liquid

was indeed embedded in the framework of supports and structural regulators, rather than loaded on the surface of the pores of solid materials. 1-ethyl-3-methylimidazolium pyrrole ionic liquid loaded on the surface of the support was easy to decompose and burn, wherein 1-ethyl-3-methylimidazolium pyrrole ionic liquid would start decomposing at 170°C in an air atmosphere, and decompose completely before 350°C.

Table 11

| Results of the carbonate transesterification catalyzed by ionic liquid embedded in the ethyl orthosilicate-acetate-based support | | | | | |
|---|---|---|---|---|---|
| Catalyst | Reaction raw material and Reaction temperature (°C) | Raw material 1 conversion efficiency (%) | Raw material 2 conversion efficiency (%) | Product 1 selectivity (%) | Product 2 selectivity (%) |
| C | EtOH/DMC=1/1 (76-78) | 57.17 | 52.69 | 78.35 | 21.65 |
| | DMC/DEC=1/1 (90-92) | 46.28 | 46.15 | 100 | - |
| D | EtOH/DMC=1/1 (76-78) | 2.81 | 2.47 | 98.44 | 1.55 |
| | DMC/DEC=1/1 (90-92) | 1.88 | 1.95 | 100 | - |

A filling weight of catalyst in the fixed bed was 4g, and the weight hourly space velocity was $1h^{-1}$. Reaction raw material 1 referred to a reactant on the left of notation '/', the raw material 2 referred to a reactant on the right of notation '/', the product 1 referred to EMC, and the product 2 referred to DEC.

Under the condition that two catalysts C and D were used in the transesterification of dimethyl carbonate and ethanol, or transesterification of dimethyl carbonate and diethyl carbonate in the fixed bed respectively, and a weight hourly space velocity of the raw materials was $1.5h^{-1}$, the reaction results were shown in Table 11. The prepared ethyl orthosilicate-magnesium acetate-zinc acetate-aluminum acetate- 1-ethyl-3-methylimidazolium pyrrole salt exhibited good catalytic activity of carbonate transesterification, but the baked catalyst had activity less than 3% thereof, fully indicating that the synthesized ionic liquid was the active center of carbonate transesterification while silica and $Mg^{2+}$, $Zn^{2+}$ and $Al^{3+}$ ions only served as support and structure regulator. The support could highly disperse 1-ethyl-3-methylimidazolium pyrrole ionic liquid to improve the dispersion of ionic liquid. Metal cations served as structure regulator and could form a variety of framework structures with four coordinations, three coordinations and two coordinations, or a mixing multi-coordination structure, which are more beneficial to increase the embedding weight of ionic liquid and the stability of immobilized ionic liquid.

**Example 16**

In Example 15, FIG. 14 showed X-ray diffraction spectrums of the catalysts in FIGS. 13D and 13C. The dried ethyl orthosilicate-magnesium acetate-zinc acetate-sodium aluminate-1-ethyl-3-methylimidazolium pyrrole salt catalyst had an amorphous structure, indicating that $Al^{3+}$, $Mg^{2+}$ and $Zn^{2+}$ ions entered the framework of $SiO_2$; and the catalyst after baking at 550°C for 3h had a structure similar to that of the catalyst after drying, without obvious peaks of MgO, ZnO or $Al_2O_3$, further indicating that $Al^{3+}$, $Mg^{2+}$ and $Zn^{2+}$ ions in the catalyst prepared with this method entered the framework of $SiO_2$.

**Example 17**

26.61mL ethyl orthosilicate and 60mL ethanol were added into a conical flask respectively, 4.99g aluminum nitrate, 4.52g sodium silicate, 3.52g ferric sulfate and 2.36g calcium chloride were further added therein and mixed, and when they were heated to 60°C, 12g N-Methyl-N-butylmorpholinium pyrrole salt ion was added therein, then 7.89g deionized water was added therein, the resulting mixture was aged at 60°C for 12 h, and washed three times with ethanol, and vacuum dried at 150°C for 3h, to obtain heterogeneous catalyst E with ionic liquid embedded. Under the condition that the catalyst was 4g, weight hourly space velocity of the reaction materials was $0.2h^{-1}$ and a molar ratio of the raw materials was 1:1, results of the three carbonate transesterifications were shown in Table 11. Effective embedding ionic liquids could also be achieved by using precursors of different metal nitrate, different metal silicate, different metal sulfate or different metal chloride, and the prepared immobilized catalysts of the ionic liquid exhibited excellent reactivity at low space velocity.

Table 12

| Results of the carbonate transesterification catalyzed by ionic liquid embedded with structure regulators of different salts | | | | | |
|---|---|---|---|---|---|
| Catalyst | Reaction raw material and Reaction temperature (°C) | Raw material 1 conversion efficiency (%) | Raw material 2 conversion efficiency (%) | Product 1 selectivity (%) | Product 2 selectivity (%) |
| E | EtOH/DMC=1/1 (76-78) | 68.62 | 58.65 | 80.39 | 19.61 |
| | DMC/DEC=1/1 (90-92) | 55.16 | 55.32 | 100 | - |
| | MeOH/DEC=1/1 (70-72) | 70.38 | 57.55 | 82.49 | 17.51 |

A filling weight of catalyst in the fixed bed was 4g, and weight hourly space velocity was $1h^{-1}$. Reaction raw material 1 referred to a reactant on the left of notation 7’, the raw material 2 referred to a reactant on the right of notation ’/’, the product 1 referred to EMC, and the product 2 referred to DEC or DMC.

The above are only a few examples of the present disclosure, and are not intended to limit the present disclosure in any way. Although the present disclosure has been described with reference to the preferred examples as above, it should be understood that the preferred examples are not intended to limit the present disclosure. Without departing from the scope of the technical solution of the present disclosure, slight changes or modifications made by any person skilled in the art, based on the technical content disclosed above, are equivalent examples and belong to the scope of the technical solution of the present disclosure. The scope of the invention is set by the appended claims.

## Claims

1. A heterogeneous catalyst, **characterized in that**, the heterogeneous catalyst comprises an ionic liquid and a support which embeds the ionic liquid in a riveting manner, wherein

   the ionic liquid comprises an anion and a cation; wherein, the cation has a structure represented by Formula I or Formula II;

   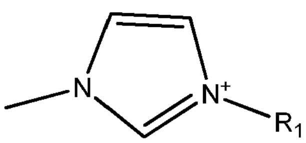 Formula I 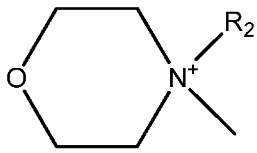 Formula II

   the anion has a structure represented by Formula III, Formula IV or Formula V;

   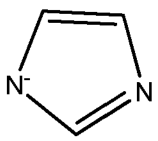 Formula III 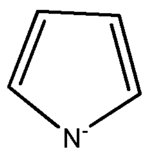 Formula IV 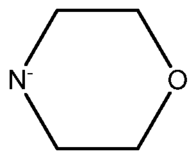 Formula V

   wherein $R_1$ and $R_2$ independently from each other are C1-C6 alkyl, C2-C6 alkenyl or C3-C6 aryl.

2. The heterogeneous catalyst according to claim 1, **characterized in that**, the ionic liquid is embedded in the framework of the support, and a weight content ratio of the active ionic liquid to the support in the heterogeneous catalyst is in a range of 1%-30%: 10%-60%.

3. The heterogeneous catalyst according to claim 2, **characterized in that**, the heterogeneous catalyst further comprises a structure regulator, and the ionic liquid is embedded in a composite framework of the support and the

structure regulator.

4. The heterogeneous catalyst according to claim 3, **characterized in that**, the structure regulator comprises at least one selected from Mg, Ca, Ba, La, Ce, Zr, Fe, Zn, Li, Cs, and Al, and a weight content of the structure regulator in the heterogeneous catalyst is in a range from 10% to 60%.

5. A method for preparing the heterogeneous catalyst according to any of claims 1-4, **characterized in that**, the method comprises the following steps:

   a) obtaining a homogeneous catalyst, wherein the homogeneous catalyst is prepared by the method comprises the following steps:

      a1) adding an alkali into solution I comprising an ionic liquid anion source, and reacting to obtain an ionic liquid anion metal salt; and
      a2) dissolving the ionic liquid anion metal salt in a solvent, adding an ionic liquid cation salt, and reacting to obtain the ionic liquid; and

   b) adding water to a mixture comprising a support precursor and an ionic liquid, and carrying out hydrolysis to obtain the heterogeneous catalyst,

   wherein

   in step a1), the ionic liquid anion source comprises imidazole, pyrrole or morpholine;
   in step a2), the ionic liquid cation salt is at least one selected from 1-$R_1$-3-methylimidazolium bromide, 1-$R_1$-3-methyl-imidazolium iodide, N-methyl-N-$R_2$-morpholinium bromide, and N-methyl-N-$R_2$-morpholinium iodide;
   in step a2), the solvent comprises a water-carrying agent which is at least one selected from ethanol, benzene, toluene and xylene.

6. The method according to claim 5, **characterized in that**,
   in step a2), the ratio of the ionic liquid anion metal salt to the solvent is in a range of 0.1-0.9: 0.05-1.2 g/mL.

7. The method according to claim 6, **characterized in that**,

   in step a1), the concentration of the ionic liquid anion source in the solution I is in a range from 0.05g/mL to 0.8 g/mL, and a molar ratio of the ionic liquid anion source to the alkali is in a range from 0.9 to 1.1, reaction conditions are as follows: the reaction temperature is in a range from 50°C to 80°C and the reaction time is in a range from 5h to 12 h;
   step a1) further comprises: after the reaction is completed, removing the solvent in the reaction system to obtain imidazole anion salt, pyrrole anion salt or morpholine anion salt;
   in step a2), reaction conditions are as follows: the reaction temperature is in a range from 25°C to 80°C and the reaction time is in a range from 12h to 48h.

8. The method according to claim 5, **characterized in that**, in step b), the support precursor comprises at least one selected from ethyl orthosilicate, tetrabutyl titanate, aluminum isopropoxide, and sodium aluminate, and a weight ratio of the support precursor, ionic liquid and water is in a range of 0.2-0.8: 0.03-0.4: 0.1-0.4.

9. The method according to claim 5, **characterized in that**, in step b), the hydrolysis is carried out at a hydrolysis temperature ranging from 25°C to 80°C, and after hydrolysis, an aging step is carried out to obtain the heterogeneous catalyst, wherein, the aging temperature is in a range from 40°C to 120°C and the aging time is in a range from 6h to 24h.

10. The method according to claim 5, **characterized in that**, in step b), the mixture comprises a structure regulator precursor which comprises at least one selected from corresponding acetate, silicate, hydrochloride, and nitrate of the structure regulator.

11. The method according to claim 10, **characterized in that**, step b) further comprises: mixing the support precursor, the structure regulator precursor with the solvent, then adding the ionic liquid therein, mixing uniformly, and then adding water to carry out hydrolysis to obtain the heterogeneous catalyst; wherein a weight ratio of the support

precursor, the structure regulator precursor, the solvent, the ionic liquid, and water is in a range of 0.2-0.8:0.05-0.4:0.3-0.8:0.03-0.4:0.1-0.4.

**12.** A method for preparing ethyl methyl carbonate by transesterification, **characterized in that**,

the catalyst is an ionic liquid catalyst or an ionic liquid-based heterogeneous catalyst; and
the conditions of the transesterification are as follows: a reaction equilibrium is reached when the reaction time is 5 min and the reaction temperature is in a range from 76°C to 78°C;
wherein, the ionic liquid comprises an anion and a cation; wherein, the cation has a structure represented by Formula I or Formula II;

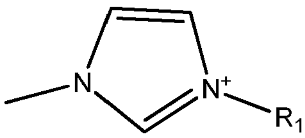 Formula I 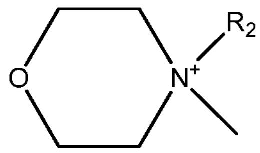 Formula II

the anion has a structure represented by Formula III, Formula IV or Formula V;

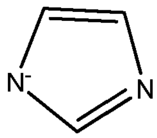 Formula III 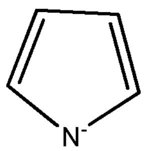 Formula IV 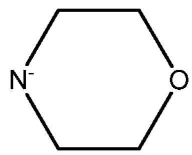 Formula V

wherein $R_1$ and $R_2$ independently from each other are C1-C6 alkyl, C2-C6 alkenyl or C3-C6 aryl; and
the ionic liquid-based heterogeneous catalyst is at least one selected from the heterogeneous catalyst according to any of claims 1-4.

**Patentansprüche**

**1.** Heterogener Katalysator, **dadurch gekennzeichnet, dass** der heterogene Katalysator eine ionische Flüssigkeit und einen Träger umfasst, der die ionische Flüssigkeit auf fesselnde Weise einbettet, wobei

die ionische Flüssigkeit ein Anion und ein Kation umfasst, wobei das Kation eine durch die Formel I oder die Formel II dargestellte Struktur aufweist;

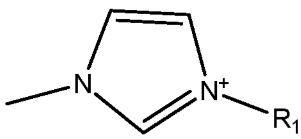 Formel I 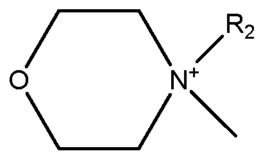 Formel II

das Anion eine durch die Formel III, die Formel IV oder die Formel V dargestellte Struktur aufweist;

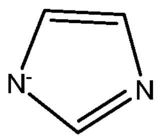 Formel III 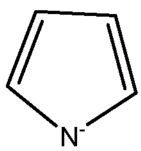 Formel IV 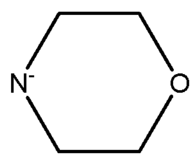 Formel V

worin $R_1$ und $R_2$ unabhängig voneinander C 1-C6-Alkyl, C2-C6-Alkenyl oder C3-C6-Aryl sind.

**2.** Heterogener Katalysator nach Anspruch 1, **dadurch gekennzeichnet, dass** die ionische Flüssigkeit in das Gerüst des Trägers eingebettet ist und das Gewichtsverhältnis der aktiven ionischen Flüssigkeit zum Träger in dem heterogenen Katalysator im Bereich von 1%-30% : 10%-60% liegt.

3. Heterogener Katalysator nach Anspruch 2, **dadurch gekennzeichnet, dass** der heterogene Katalysator ferner einen Strukturregulator umfasst und die ionische Flüssigkeit in ein Verbundgerüst aus dem Träger und dem Strukturregulator eingebettet ist.

4. Heterogener Katalysator nach Anspruch 3, **dadurch gekennzeichnet, dass** der Strukturregulator mindestens eines, ausgewählt aus Mg, Ca, Ba, La, Ce, Zr, Fe, Zn, Li, Cs und Al, umfasst, und dass der Gewichtsanteil des Strukturregulators in dem heterogenen Katalysator im Bereich von 10% bis 60% liegt.

5. Verfahren zur Herstellung des heterogenen Katalysators nach einem der Ansprüche 1-4, **dadurch gekennzeichnet, dass** das Verfahren die folgenden Schritte umfasst:

   a) Erhalten eines homogenen Katalysators, wobei der homogene Katalysator durch das Verfahren hergestellt wird, das die folgenden Schritte umfasst:

      a1) Zugeben eines Alkalis zu einer Lösung I, die eine Anionenquelle der ionischen Flüssigkeit enthält, und Umsetzen, um ein anionisches Metallsalz der ionischen Flüssigkeit zu erhalten; und
      a2) Lösen des anionischen Metallsalzes der ionischen Flüssigkeit in einem Lösungsmittel, Zugeben eines kationischen Salzes der ionischen Flüssigkeit und Umsetzen, um die ionische Flüssigkeit zu erhalten; und

   b) Zugeben von Wasser zu einer Mischung, die einen Träger-Vorläufer und eine ionische Flüssigkeit umfasst, und Durchführen einer Hydrolyse, um den heterogenen Katalysator zu erhalten,

   wobei

   in Schritt a1) die Anionenquelle der ionischen Flüssigkeit Imidazol, Pyrrol oder Morpholin umfasst;
   in Schritt a2) das kationische Salz der ionischen Flüssigkeit mindestens eines ist, ausgewählt aus 1-$R_1$-3-Methylimidazoliumbromid, 1-$R_1$-3-Methyl-imidazoliumiodid, N-Methyl-N-$R_2$-morpholiniumbromid und N-Methyl-N-$R_2$-morpholiniumiodid;
   in Schritt a2) das Lösungsmittel ein wassertragendes Mittel umfasst, das mindestens eines ist, ausgewählt aus Ethanol, Benzol, Toluol und Xylol.

6. Verfahren nach Anspruch 5, **dadurch gekennzeichnet, dass** in Schritt a2) das Verhältnis des anionischen Metallsalzes der ionischen Flüssigkeit zum Lösungsmittel in einem Bereich von 0,1~0,9 : 0,05-1,2 g/ml liegt.

7. Verfahren nach Anspruch 6, **dadurch gekennzeichnet, dass**

   in Schritt a1) die Konzentration der Anionenquelle der ionischen Flüssigkeit in der Lösung I im Bereich von 0,05 g/ml bis 0,8 g/ml liegt und das Molverhältnis der Anionenquelle der ionischen Flüssigkeit zum Alkali im Bereich von 0,9 bis 1, 1 liegt, die Reaktionsbedingungen wie folgt sind: die Reaktionstemperatur liegt im Bereich von 50°C bis 80°C und die Reaktionszeit liegt im Bereich von 5 h bis 12 h;
   Schritt a1) ferner umfasst: nachdem die Umsetzung abgeschlossen ist, Entfernen des Lösungsmittels im Reaktionssystem, um ein Imidazol-Anionensalz, ein Pyrrol-Anionensalz oder ein Morpholin-Anionensalz zu erhalten;
   in Schritt a2) die Reaktionsbedingungen wie folgt sind: die Reaktionstemperatur liegt im Bereich von 25°C bis 80°C und die Reaktionszeit liegt im Bereich von 12 h bis 48 h.

8. Verfahren nach Anspruch 5, **dadurch gekennzeichnet, dass** in Schritt b) der Träger-Vorläufer mindestens eines, ausgewählt aus Ethylorthosilikat, Tetrabutyltitanat, Aluminiumisopropoxid und Natriumaluminat, umfasst und das Gewichtsverhältnis des Träger-Vorläufers, der ionischen Flüssigkeit und des Wassers im Bereich von 0,2~0,8 : 0,03~0,4 : 0,1~0,4 liegt.

9. Verfahren nach Anspruch 5, **dadurch gekennzeichnet, dass** in Schritt b) die Hydrolyse bei einer Hydrolysetemperatur im Bereich von 25°C bis 80°C durchgeführt wird und nach der Hydrolyse ein Alterungsschritt durchgeführt wird, um den heterogenen Katalysator zu erhalten, wobei die Alterungstemperatur im Bereich von 40°C bis 120°C liegt und die Alterungszeit im Bereich von 6 h bis 24 h liegt.

10. Verfahren nach Anspruch 5, **dadurch gekennzeichnet, dass** das Gemisch in Schritt b) einen Strukturregulator-Vorläufer umfasst, der mindestens eines, ausgewählt aus dem entsprechenden Acetat, Silikat, Hydrochlorid und

Nitrat des Strukturregulators, umfasst.

**11.** Verfahren nach Anspruch 10, **dadurch gekennzeichnet, dass** Schritt b) ferner umfasst:
Mischen des Träger-Vorläufers, des Strukturregulator-Vorläufers mit dem Lösungsmittel, dann Zugeben der ionischen Flüssigkeit darin, gleichmäßiges Mischen und dann Zugeben von Wasser, um die Hydrolyse durchzuführen, um den heterogenen Katalysator zu erhalten; wobei das Gewichtsverhältnis des Träger-Vorläufers, des Strukturregulator-Vorläufers, des Lösungsmittels, der ionischen Flüssigkeit und des Wassers im Bereich von 0,2~0,8 : 0,05~0,4 : 0,3~0,8 : 0,03~0,4 : 0,1~0,4 liegt.

**12.** Verfahren zur Herstellung von Ethylmethylcarbonat durch Umesterung, **dadurch gekennzeichnet, dass**

der Katalysator ein ionischer Flüssigkeitskatalysator oder ein heterogener Katalysator auf Basis einer ionischen Flüssigkeit ist; und
die Bedingungen der Umesterung wie folgt sind: ein Reaktionsgleichgewicht wird erreicht, wenn die Reaktionszeit 5 min beträgt und die Reaktionstemperatur im Bereich von 76°C bis 78°C liegt;
wobei die ionische Flüssigkeit ein Anion und ein Kation umfasst, wobei das Kation eine durch die Formel I oder die Formel II dargestellte Struktur aufweist;

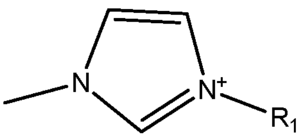
Formel I

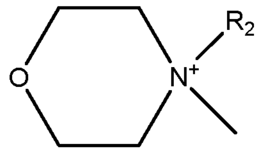
Formel II

das Anion eine durch die Formel III, die Formel IV oder die Formel V dargestellte Struktur aufweist;

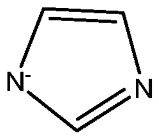
Formel III

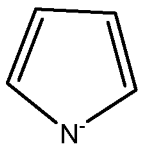
Formel IV

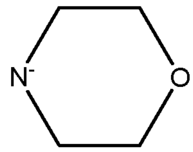
Formel V

worin $R_1$ und $R_2$ unabhängig voneinander C 1-C6-Alkyl, C2-C6-Alkenyl oder C3-C6-Aryl sind; und
der heterogene Katalysator auf Basis einer ionischen Flüssigkeit mindestens einer ist, ausgewählt aus dem heterogenen Katalysator nach einem der Ansprüche 1-4.

## Revendications

**1.** Catalyseur hétérogène, **caractérisé en ce que** le catalyseur hétérogène comporte un liquide ionique et un support qui incorpore le liquide ionique d'une manière rivetée, dans lequel

le liquide ionique comporte un anion et un cation ; dans lequel le cation a une structure représentée par la Formule I ou la Formule II ;

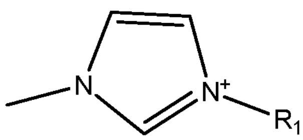
Formule I

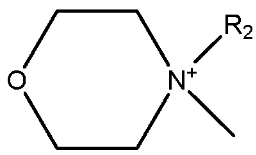
Formule II

l'anion a une structure représentée par la Formule III, la Formule IV ou la Formule V;

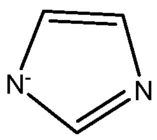 Formule III 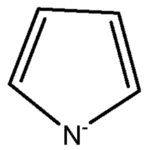 Formule IV 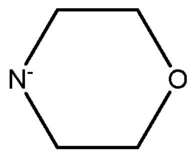 Formule V

dans lesquelles $R_1$ et $R_2$ sont, indépendamment l'un de l'autre, un alkyle en C1 à C6, un alcényle en C2 à C6 ou un aryle en C3 à C6.

**2.** Catalyseur hétérogène selon la revendication 1, **caractérisé en ce que** le liquide ionique est incorporé dans l'ossature du support et un rapport de teneur en poids du liquide ionique actif sur le support dans le catalyseur hétérogène est dans une plage de 1 % à 30 %: 10 % à 60 %.

**3.** Catalyseur hétérogène selon la revendication 2, **caractérisé en ce que** le catalyseur hétérogène comporte en outre un régulateur de structure, et le liquide ionique est incorporé dans une ossature composite constituée du support et du régulateur de structure.

**4.** Catalyseur hétérogène selon la revendication 3, **caractérisé en ce que** le régulateur de structure comporte au moins un élément choisi parmi Mg, Ca, Ba, La, Ce, Zr, Fe, Zn, Li, Cs et Al, et une teneur en poids du régulateur de structure dans le catalyseur hétérogène est dans une plage de 10 % à 60 %.

**5.** Procédé pour préparer le catalyseur hétérogène selon l'une quelconque des revendications 1 à 4, **caractérisé en ce que** le procédé comporte les étapes suivantes consistant à :

a) obtenir un catalyseur homogène, dans lequel le catalyseur homogène est préparé par le procédé comportant les étapes suivantes :

a1) ajouter un alcali dans une solution I comportant une source d'anions de liquide ionique et faire réagir pour obtenir un sel métallique d'anions de liquide ionique ; et
a2) dissoudre le sel métallique d'anions de liquide ionique dans un solvant, ajouter un sel de cations de liquide ionique et faire réagir pour obtenir le liquide ionique ; et

b) ajouter de l'eau à un mélange comportant un précurseur de support et un liquide ionique, et effectuer une hydrolyse pour obtenir le catalyseur hétérogène,

dans lequel

à l'étape a1), la source d'anions de liquide ionique comporte un imidazole, un pyrrole ou une morpholine ;
à l'étape a2), le sel de cations de liquide ionique est au moins un élément choisi parmi du bromure de 1-$R_1$-3-méthyl-imidazolium, de l'iodure de 1-$R_1$-3-méthyl-imidazolium, du bromure de N-méthyl-N-$R_2$-morpholinium et de l'iodure de N-méthyl-N-$R_2$-morpholinium ;
à l'étape a2), le solvant comporte un agent porteur d'eau qui est au moins un élément choisi parmi l'éthanol, le benzène, le toluène et le xylène.

**6.** Procédé selon la revendication 5, **caractérisé en ce que**
à l'étape a2), le rapport du sel métallique d'anions de liquide ionique sur le solvant est dans une plage de 0,1 à 0,9: 0,05 à 1,2 g/mL.

**7.** Procédé selon la revendication 6, **caractérisé en ce que**

à l'étape a1), la concentration de la source d'anions de liquide ionique dans la solution I est dans une plage de 0,05 g/mL à 0,8 g/mL, et un rapport molaire de la source d'anions de liquide ionique sur l'alcali est dans une plage de 0,9 à 1,1, les conditions de réaction étant les suivantes : la température de réaction est dans une plage de 50 °C à 80 °C et la durée de réaction est dans une plage de 5 h à 12 h ;
l'étape a1) comporte en outre de, après la fin de la réaction, éliminer le solvant dans le système de réaction pour obtenir un sel d'anions d'imidazole, un sel d'anions de pyrrole ou un sel d'anions de morpholine ;
à l'étape a2), les conditions de réaction sont les suivantes : la température de réaction est dans une plage de 25 °C à 80 °C et la durée de réaction est dans une plage de 12 h à 48 h.

**8.** Procédé selon la revendication 5, **caractérisé en ce que**, à l'étape b), le précurseur de support comporte au moins un élément choisi parmi un orthosilicate d'éthyle, un titanate de tétrabutyle, un isopropoxyde d'aluminium et un aluminate de sodium, et un rapport pondéral du précurseur de support, du liquide ionique et de l'eau est dans une plage de 0,2 à 0,8: 0,03 à 0,4: 0,1 à 0,4.

**9.** Procédé selon la revendication 5, **caractérisé en ce que**, à l'étape b), l'hydrolyse est effectuée à une température d'hydrolyse variant de 25 °C à 80 °C, et après l'hydrolyse, une étape de vieillissement est réalisée pour obtenir le catalyseur hétérogène, dans lequel la température de vieillissement est dans une plage de 40 °C à 120 °C et la durée de vieillissement est dans une plage de 6 h à 24 h.

**10.** Procédé selon la revendication 5, **caractérisé en ce que**, à l'étape b), le mélange comporte un précurseur de régulateur de structure qui comporte au moins un élément choisi parmi un acétate, un silicate, un hydrochlorure et un nitrate correspondant du régulateur de structure.

**11.** Procédé selon la revendication 10, **caractérisé en ce que** l'étape b) comporte en outre de :
mélanger le précurseur de support et le précurseur de régulateur de structure avec le solvant, puis ajouter le liquide ionique dans ceux-ci, mélanger de façon uniforme et ajouter ensuite de l'eau pour réaliser l'hydrolyse pour obtenir le catalyseur hétérogène ; dans lequel un rapport pondéral du précurseur de support, du précurseur de régulateur de structure, du solvant, du liquide ionique et de l'eau est dans une plage de 0,2 à 0,8:0,05 à 0,4:0,3 à 0,8:0,03 à 0,4:0,1 à 0,4.

**12.** Procédé pour préparer du carbonate d'éthyl méthyle par transestérification, **caractérisé en ce que**

le catalyseur est un catalyseur de liquide ionique ou un catalyseur hétérogène à base de liquide ionique ; et
les conditions de la transestérification sont les suivantes : un équilibre de réaction est atteint lorsque la durée de réaction est de 5 min et la température de réaction est dans une plage de 76 °C à 78 °C ;
dans lequel le liquide ionique comporte un anion et un cation ; dans lequel le cation a une structure représentée par la Formule I ou la Formule II ;

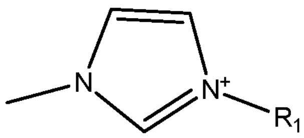 Formule I 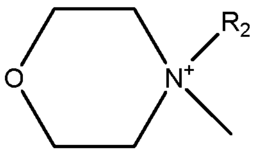 Formule II

l'anion a une structure représentée par la Formule III, la Formule IV ou la Formule V;

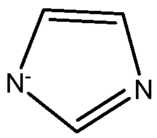 Formule III 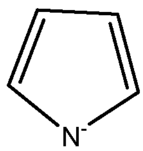 Formule IV 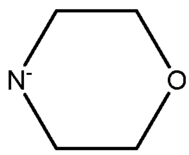 Formule V

dans lesquelles $R_1$ et $R_2$ sont, indépendamment l'un de l'autre, un alkyle en C1 à C6, un alcényle en C2 à C6 ou un aryle en C3 à C6 ; et
le catalyseur hétérogène à base de liquide ionique est au moins un élément choisi parmi le catalyseur hétérogène selon l'une quelconque des revendications 1 à 4.

# ACCOMPANYING DRAWINGS

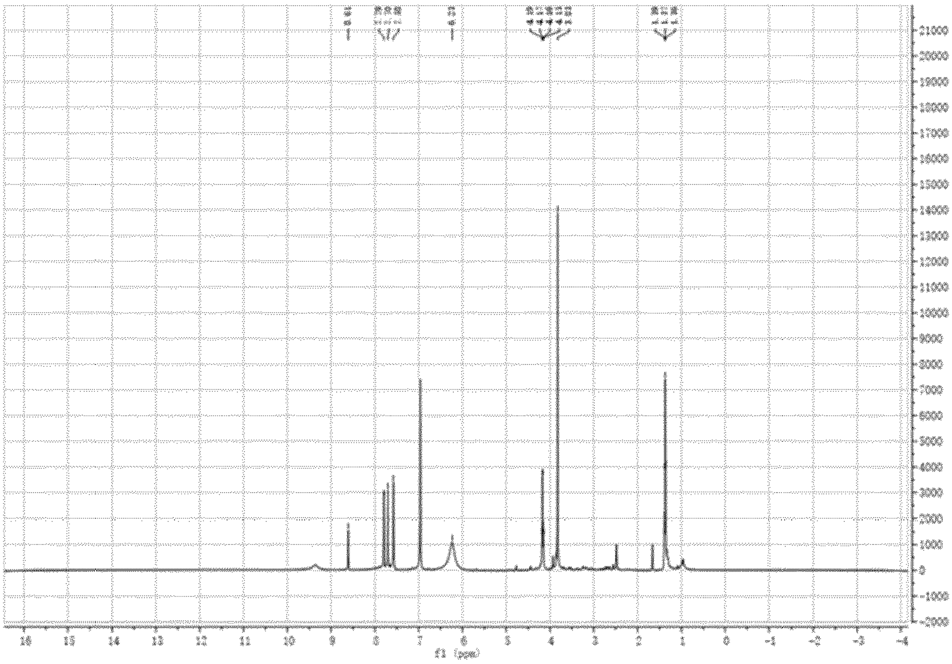

FIG.1

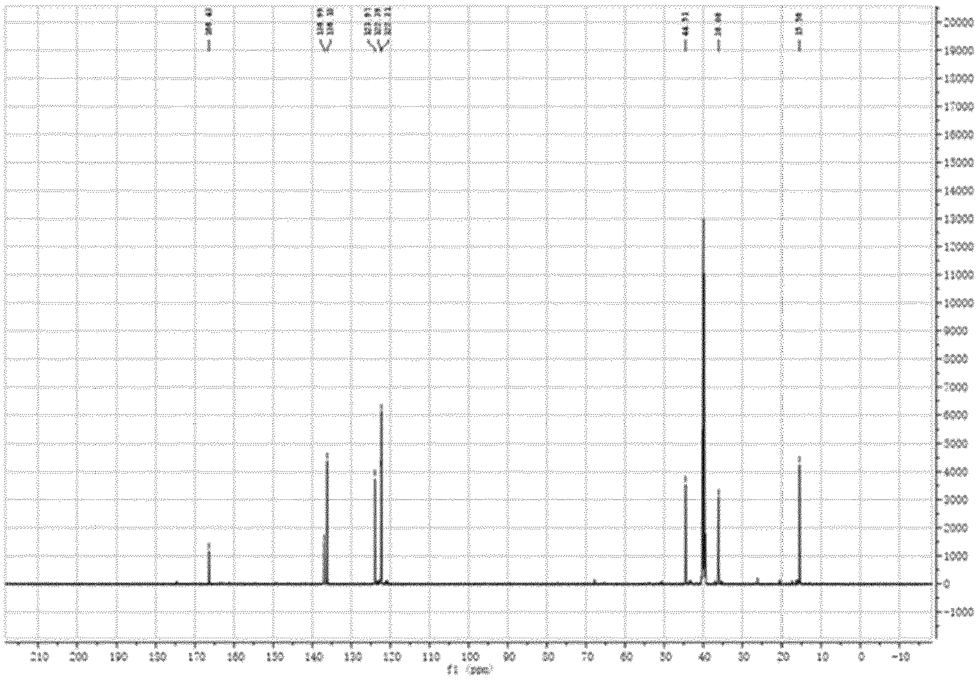

FIG.2

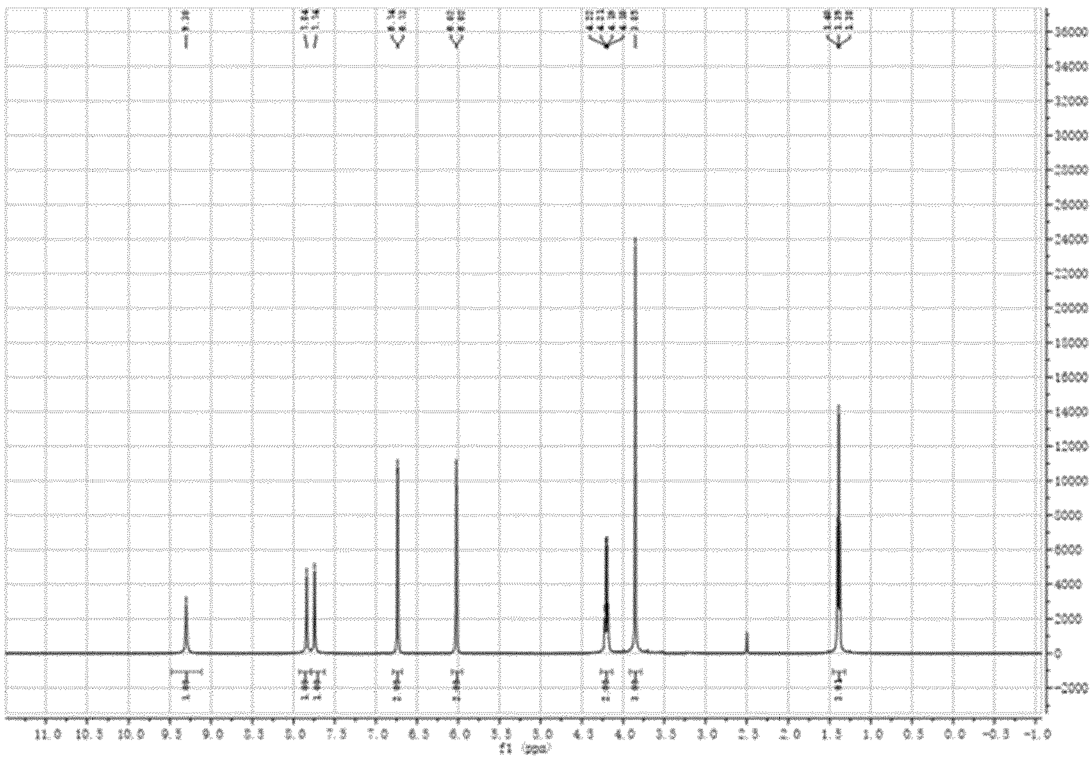

FIG.3

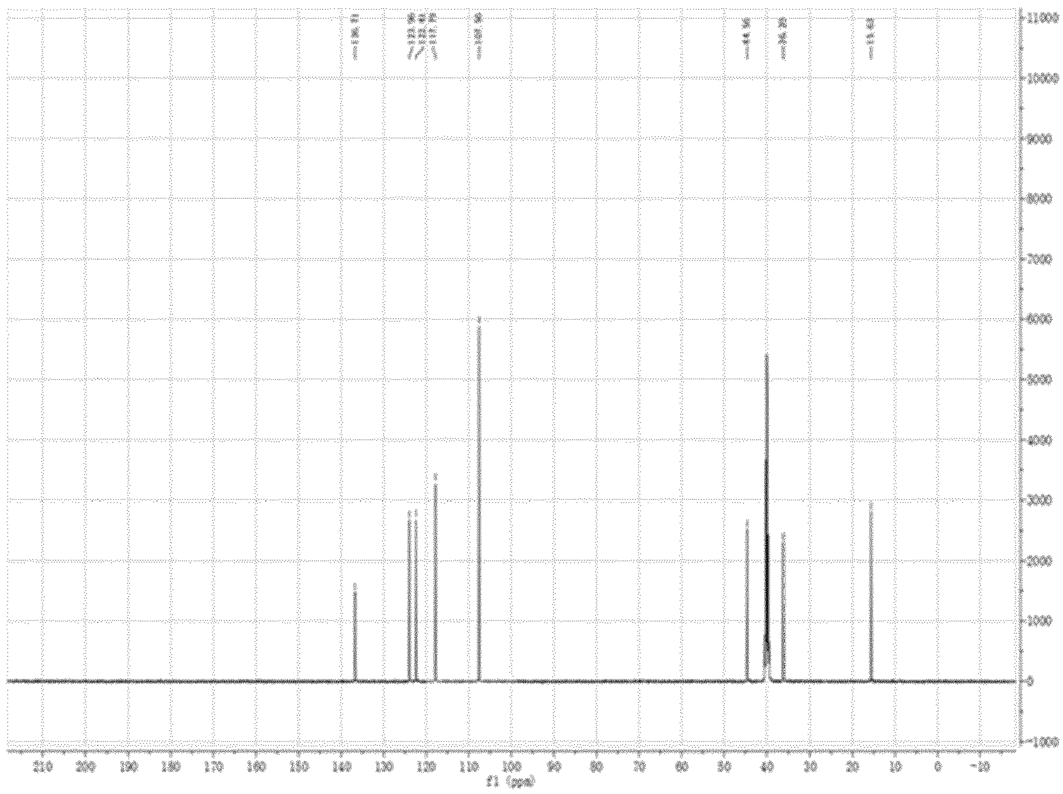

FIG.4

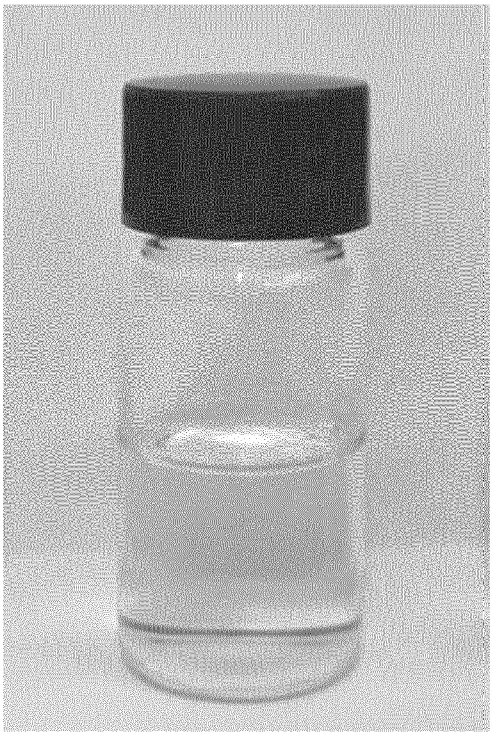

FIG.5

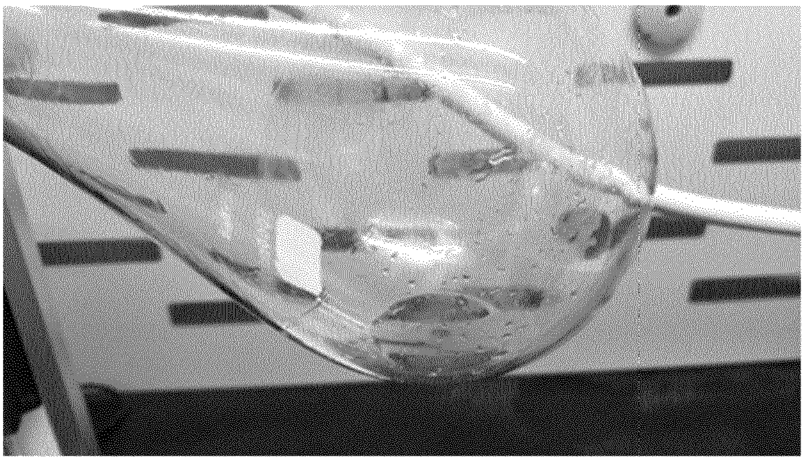

FIG.6

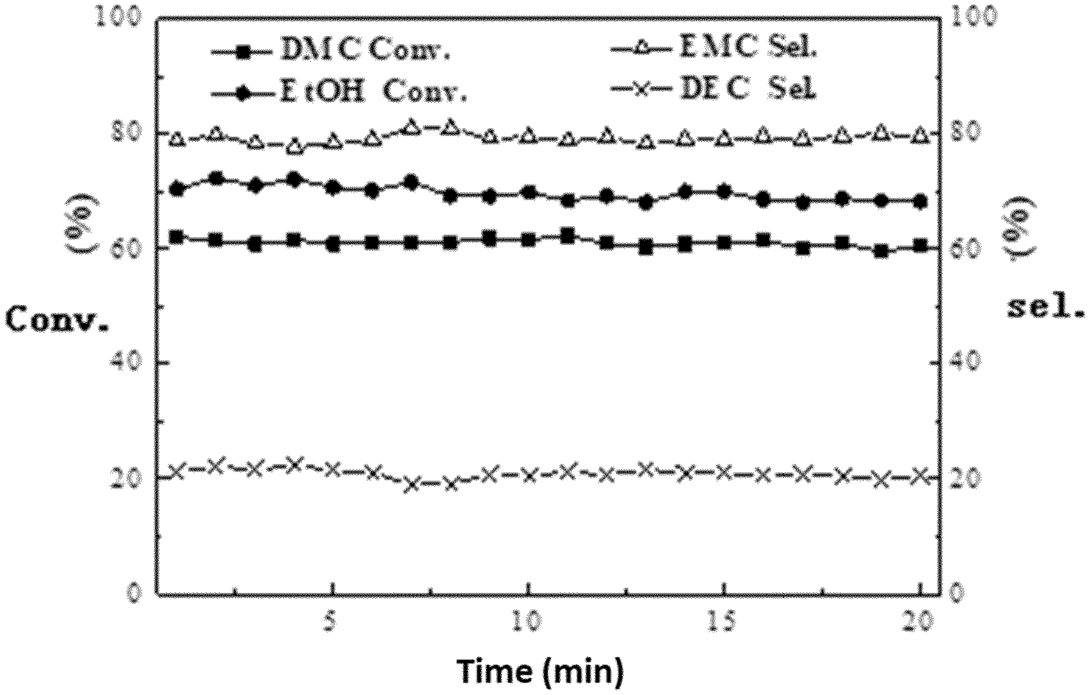
DMC Conv.
EtOH Conv.
EMC Sel.
DEC Sel.
Conv. (%)
sel. (%)
Time (min)
0
20
40
60
80
100
5
10
15
20

FIG.7

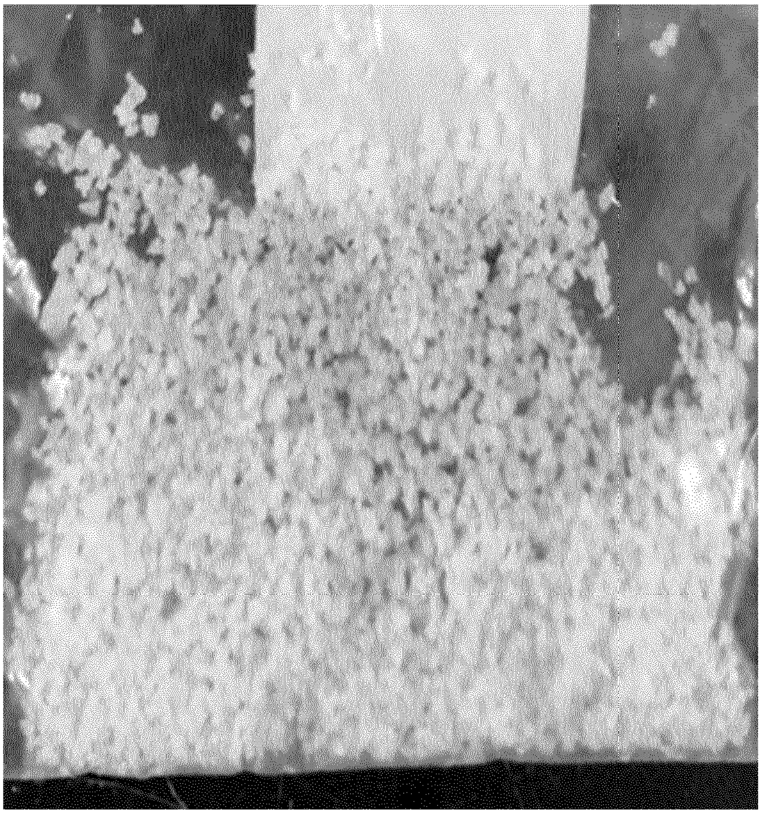

FIG.8

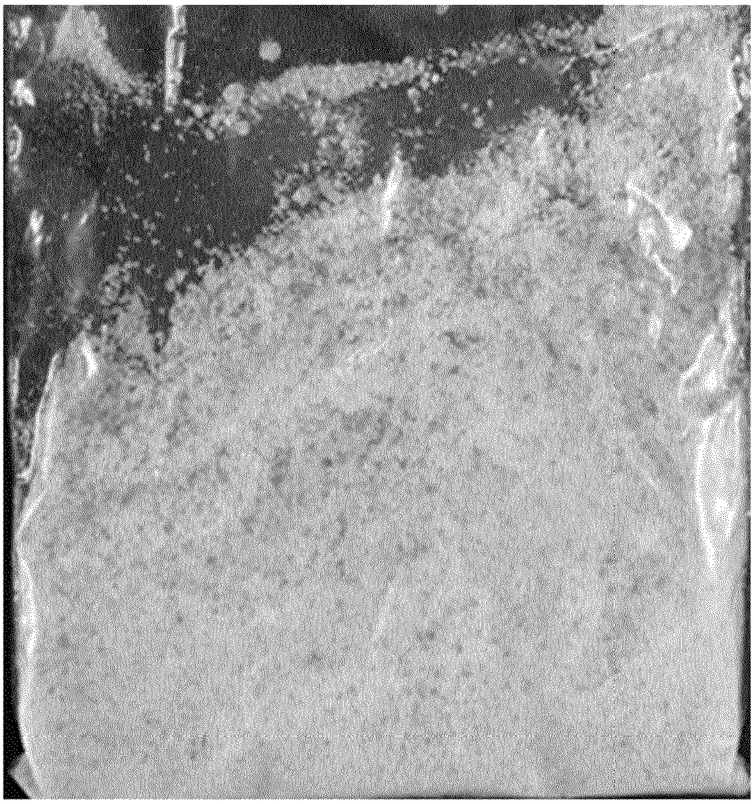

FIG.9

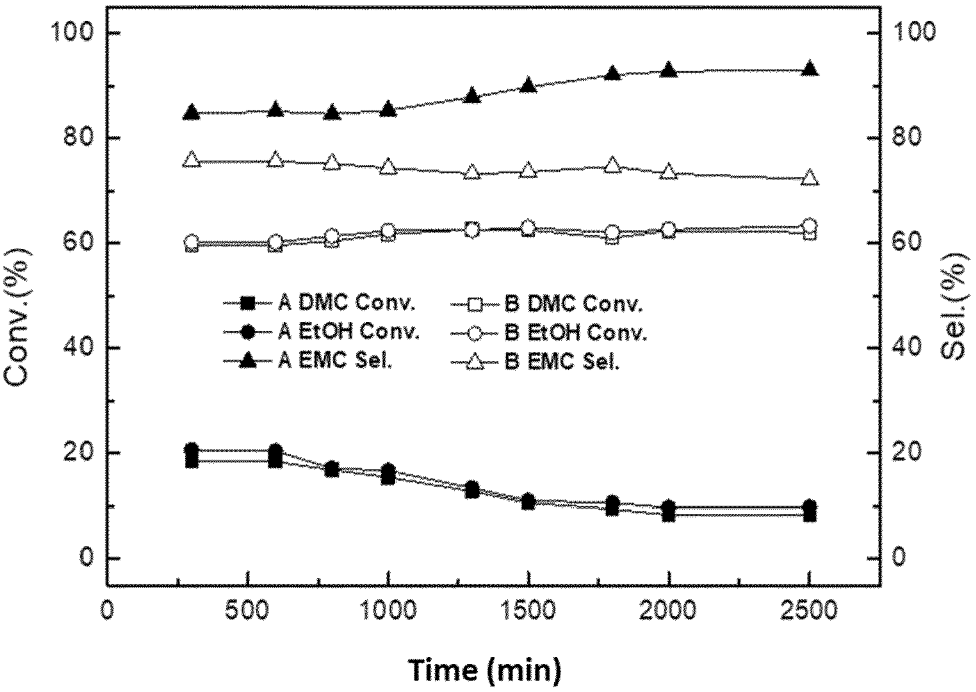
Conv.(%)
Sel.(%)
Time (min)
A DMC Conv.
B DMC Conv.
A EtOH Conv.
B EtOH Conv.
A EMC Sel.
B EMC Sel.
0
20
40
60
80
100
500
1000
1500
2000
2500

FIG.10

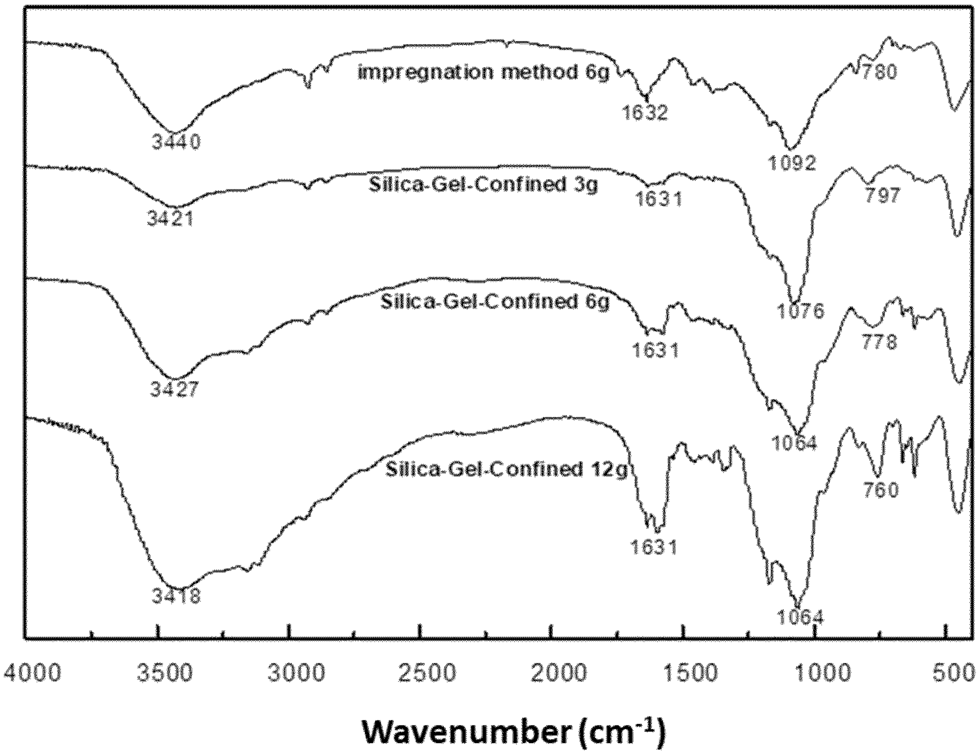
impregnation method 6g
3440
1632
1092
780
Silica-Gel-Confined 3g
3421
1631
1076
797
Silica-Gel-Confined 6g
3427
1631
1064
778
Silica-Gel-Confined 12g
3418
1631
1064
760
4000
3500
3000
2500
2000
1500
1000
500
Wavenumber (cm-1)

FIG.11

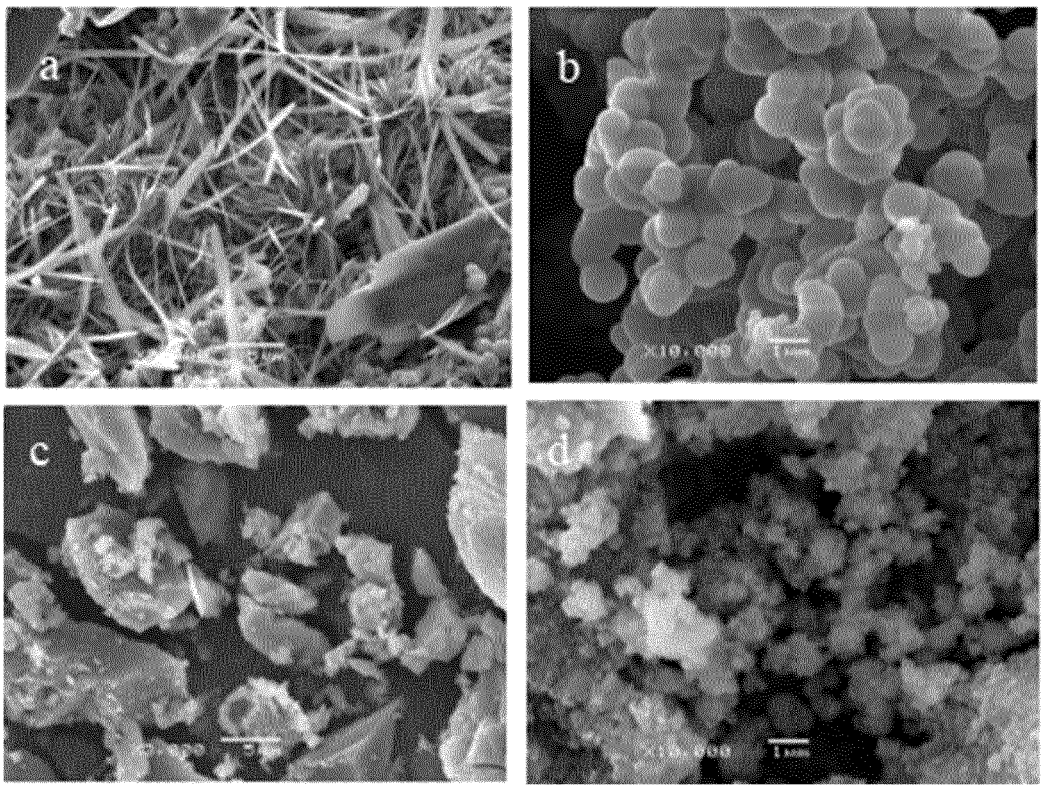
a
b
c
d

FIG.12

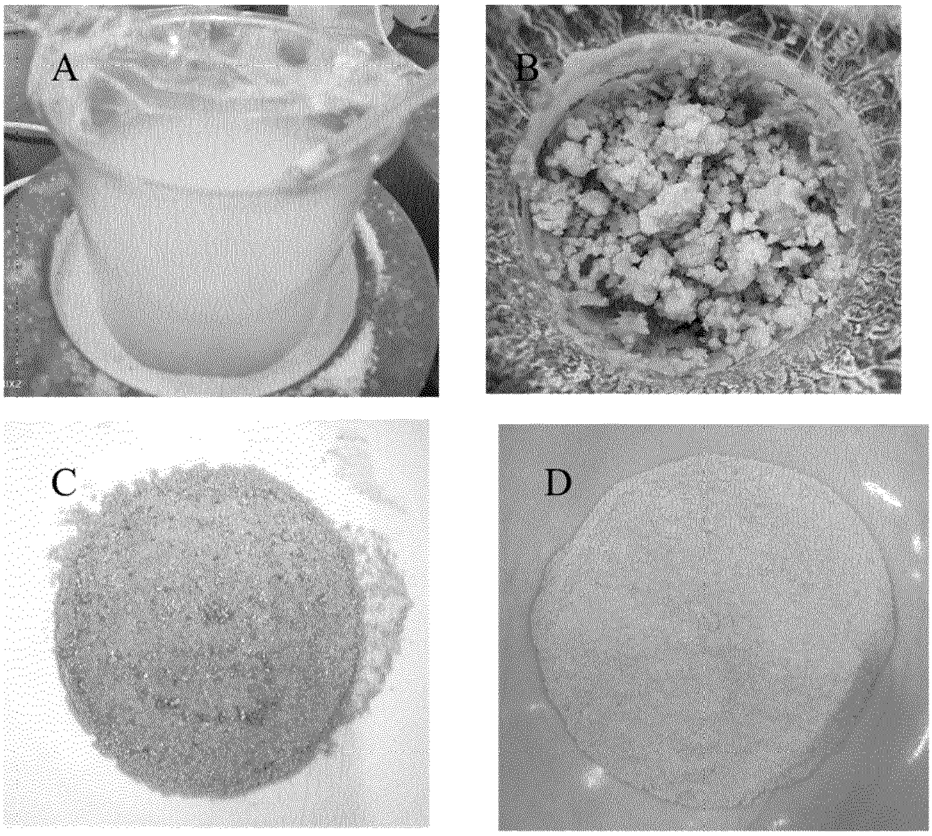


FIG.13

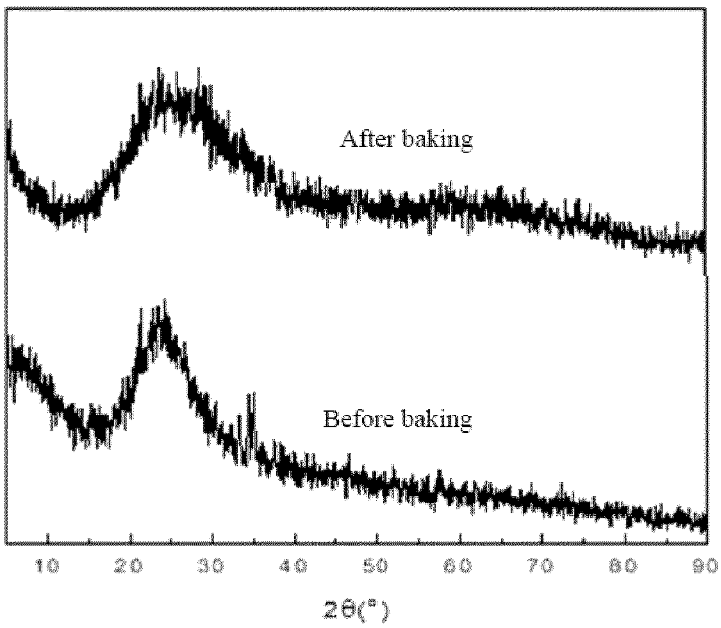


FIG.14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103521263 A
- CN 108117536 A
- CN 107162879 A
- CN 102863339 B
- CN 106582813 A


### Non-patent literature cited in the description


- **CHEN XUEWEI et al.** Solvent-free aza-Markovnikov and aza-Michael additions promoted by a catalytic amount of imidazolide basic ionic liquids. *Tetrahedron letters,* vol. 52 (28), 3588-3591
- **PAN SU-JUAN et al.** Synthesis of di-2-ethy hexyl carbonate using [Bmim]Im as a catalyst. *DALIAN GONGYE DAXUE XUEBAO,* 2011, vol. 30 (2), ISSN 1674-1404, 129-132